# FASCICULE DE BREVET EUROPEEN

(11) **EP 0 570 263 B1**
(45) Date de publication et mention de la délivrance du brevet: **03.12.1997**
(21) Numéro de dépôt: 93401174.3
(22) Date de dépôt: 07.05.1993
(51) Int. Cl.: C07C 311/19, A61K 31/19, C07C 311/20, C07D 211/34

(54) **Nouveaux derivés d'acides alcenecarboxyliques, leur procédé de préparation et les compositions pharmaceutiques qui les contiennent**
Neue Alkencarbonsäurederivate, Verfahren zu ihrer Herstellung und sie enthaltende pharmazeutische Zusammensetzungen
New alkene carboxylic acid derivatives, process for their preparation and pharmaceutic compositions containing them

(30) Priorité: 15.05.1992 FR 9205905
(43) Date de publication de la demande: 18.11.1993
(73) Titulaire: ADIR ET COMPAGNIE, 92415 Courbevoie Cédex (FR)
(72) Inventeur: Lavielle, Gilbert, F-78170 La Celle Saint Cloud (FR); Hautefaye, Patrick, F-77170 Servon Brie Comte Robert (FR); Laubie, Michel, F-92420 Vaucresson (FR); Verbeuren, Tony, F-78540 Vernouillet (FR)

(56) Documents cités:
- EP-A- 0 312 906
- EP-A- 0 384 747
- EP-A- 0 472 449
- US-A- 4 965 279
- JOURNAL OF MEDICINAL CHEMISTRY vol. 34, no. 6, Juin 1991, WASHINGTON US pages 1885 - 1891 M. TAKASUKA ET AL.
- Exp.Opin.Invest.Drug (1994) 3 (5):469-480

## Description

La présente invention concerne de nouveaux dérivés d'acides alcène-carboxyliques, leur procédé de préparation et les compositions pharmaceutiques qui les contiennent.

Plus particulièrement, les composés décrits dans la présente invention possèdent des propriétés antithromboxane A₂ aussi bien en tant qu'antagonistes des récepteurs au thromboxane A₂ (TXA₂) qu'en tant qu'inhibiteurs de l'activité de l'enzyme responsable de la synthèse du thromboxane A₂ : la thromboxane A₂-synthase.

Le thromboxane A₂ est un métabolite de l'acide arachidonique produit par les plaquettes sanguines qui provoque une constriction importante des vaisseaux sanguins et induit l'agrégation des plaquettes. La production du thromboxane A₂ est augmentée dans des maladies comme l'angine de poitrine ou l'accident vasculaire cérébral et il joue un rôle très important dans tous les processus conduisant aux maladies thrombotiques.

Il était donc particulièrement intéressant de synthétiser des substances susceptibles d'inhiber les activités proagrégantes et vasoconstrictives du thromboxane A₂ soit en tant qu'antagonistes des récepteurs au thromboxane A₂, soit en tant qu'inhibiteurs de la thromboxane A₂-synthase.

Des dérivés d'acides alcènecarboxyliques possédant des propriétés antithrombotiques ont été décrits dans la littérature. C'est le cas, en particulier, des composés décrits dans les brevets EP 405 391, EP 312 906 ou bien encore EP 472 449.
Les composés décrits dans la présente invention, outre le fait qu'ils soient nouveaux, possèdent des propriétés pharmacologiques nettement plus intenses que celles des autres composés décrits dans l'Art Antérieur.

Ils sont donc utiles en tant qu'antagonistes au thromboxane A₂ et en tant qu'inhibiteurs de la thromboxane A₂-synthase dans le traitement ou la prévention des maladies thrombotiques comme l'accident vasculaire. Ces antagonistes au thromboxane A₂ possèdent également des propriétés protectrices de la paroi gastrique (M.L. OGLETREE et coll., J. Pharm. and Exp. Therap., 263 (1), 374-380).

Plus spécifiquement, la présente invention concerne les composés de formule (I) 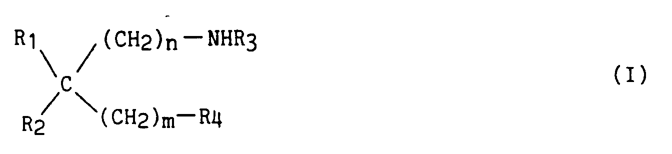 dans laquelle :
- R₁ ou R₂: identiques ou différents représentent un radical alkyle (C₁-C₆) linéaire ou ramifié, un radical phényle (substitué ou non par un ou plusieurs atomes d'halogène ou radical alkyle (C₁-C₆) linéaire ou ramifié, alkoxy (C₁-C₆) linéaire ou ramifié, hydroxy, trihalogénométhyle), un radical pyridinyl, un radical thiényl,
ou forment, avec l'atome de carbone auxquels ils sont attachés, un cycle cycloalkyle (C₄-C₇) (substitué ou non par un radical alkyle (C₁-C₆) linéaire ou ramifié), un cycle benzocycloalkyle (C₄-C₇), ou un cycle pipéridin-4-yl (substitué ou non sur l'azote de la pipéridine par groupement phénylsulfonyl lui-même éventuellement substitué par un ou plusieurs atomes d'halogène ou groupements alkyle),
- R₃: représente
- un radical phénylsulfonyl substitué ou non sur le noyau phényle par un atome d'halogène ou radical alkyle (C₁-C₆) linéaire ou ramifié,
- un radical naphtylsulfonyl,
- un radical alkyle (C₁-C₆) linéaire ou ramifié,
- un radical alkylaminocarbonyl,
- ou un radical acyle (C₁-C₆) linéaire ou ramifié,
- R₄: représente l'un quelconque des radicaux :
―CH=CH―(CH₂)ₚ―CO₂H ou ―CH₂―CH₂―(CH₂)ₚ―CO₂H
dans lesquels p est égal à 0, 1, 2 ou 3,
n et m, identiques ou différents, représentent 0, 1 ou 2,
leurs isomères, énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

Parmi les bases pharmaceutiquement acceptables, on peut citer à titre non limitatif l'hydroxyde de sodium, l'hydroxyde de potassium, la tertbutylamine, la diéthylamine, l'éthylènediamine...

Lorsque les composés de formule (I) possèdent un radical R₄=-CH=CH-(CH₂)ₚ-CO₂H, les composés préférés de l'invention sont ceux pour lesquels la configuration de la double liaison du radical R₄ est cis.

L'invention s'étend également au procédé de préparation des composés de formule (I) caractérisé en ce que l'on utilise comme produit de départ :
1/ un nitrile de formule (II) : 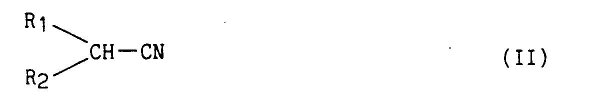 dans laquelle R₁ et R₂ ont la même signification que dans la formule (I),
   que l'on fait réagir :
   sur le dérivé bromé de formule (III) en présence de diisopropylamidure de lithium dans du tetrahydrofurane : 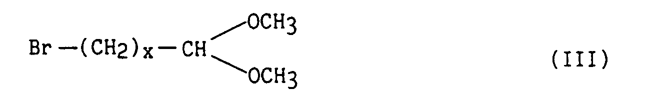 dans laquelle x représente n, n-1 ou m selon le dérivé de formule (I) que l'on souhaite obtenir,
   pour conduire au dérivé de formule (IV) : 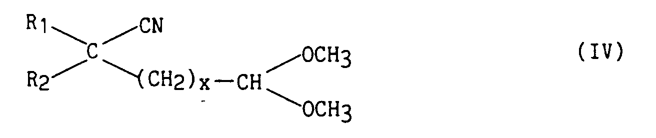 dans laquelle x a la même signification que précédemment,
   - soit: que l'on réduit à l'aide de quatre équivalents d'hydrure de lithium aluminium dans de l'éther pour conduire au composé de formule (V) : 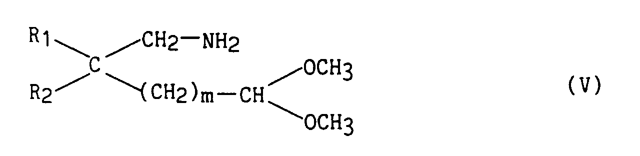 dans laquelle m a la même signification que dans la formule (I),
   que l'on transforme en composé de formule (VII) : 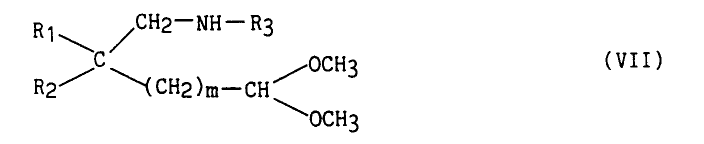 dans laquelle R₁, R₂ et m ont la même signification que dans la formule (I),
   par réaction, selon la nature de R₃ :
   * avec un dérivé halogéné de formule (VI) en présence de triéthylamine dans du toluène :

      R'₃X (VI)

      dans laquelle X représente un atome d'halogène,
      R'₃ représente un radical phénylsulfonyl substitué ou non, ou un radical acyle,
   * avec un isocyanate de formule R''-N=C=O (VI') dans laquelle R'' représente un radical alkyle et dans ce cas le radical R₃ de la formule (VII) est un radical alkylaminocarbonyl,
   * un aldéhyde de formule R''₃ CHO (VI'') dans laquelle R''₃ est un radical alkyle pour former une imine qui est ensuite réduite en amine correspondante,
   composé de formule (VII) :
   ou bien que l'on transforme en aldéhyde de formule (VIIIa) en présence d'acide formique : 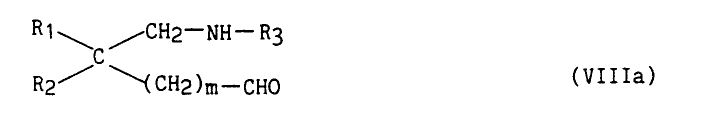 dans laquelle R₁, R₂ et m ont la même signification que dans la formule (I),
   ou bien, lorsque m représente 1 ou 2, que l'on cyclise au reflux du toluène, en composé de formule (VIIIb), en présence d'acide paratoluène sulfonique : 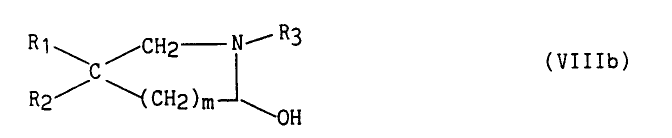 dans laquelle R₁, R₂, R₃ et m ont la même signification que précédemment,
   composé de formule (VIIIa) ou (VIIIb), que l'on fait réagir avec l'ylure de phosphore de formule (IX), préparé par réaction du sel de phosphonium correspondant en présence de terbutanolate de potassium dans du tétrahydrofurane,

   (C₆H₅)₃P=CH―(CH₂)ₚ―CO₂H (IX)

   dans laquelle p a la même signification que dans la formule (I),
   pour conduire au composé de formule (I/a), cas particulier des composés de formule (I) : 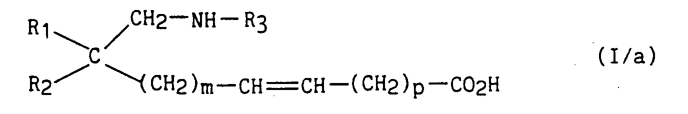
   - soit: que l'on transforme en aldéhyde correspondant de formule (X) à l'aide de trois équivalents d'hydrure de diisobutylaluminium dans du toluène, 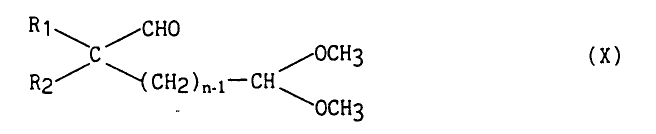 dans laquelle n a la même signification que dans la formule (I),
   que l'on fait réagir avec l'ylure de phosphore de formule (IX) défini précédemment,
   pour conduire après traitement au diazomèthane au composé de formule (XI) : 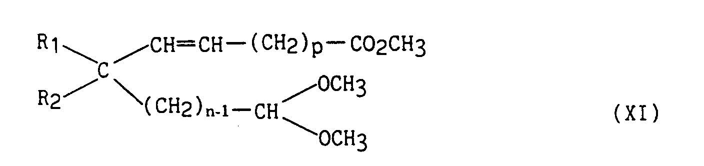 dans laquelle R₁, R₂, p et n ont la même signification que précédemment,
   qui, après hydrolyse de l'acétal, réduction de l'aldéhyde formé en alcool, et formation de l'amine correspondante, est transformé en composé de formule (I/b), cas particulier des composés de formule (I), en faisant réagir soit le dérivé halogéné de formule (VI), soit l'isocyanate de formule (VI'), soit l'aldéhyde de formule (VI'') selon la nature du radical R₃ que l'on souhaite obtenir, et en saponifiant l'ester obtenu, 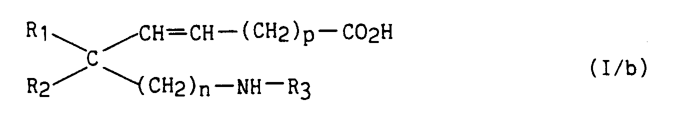
2/ un ester de formule (XII) : 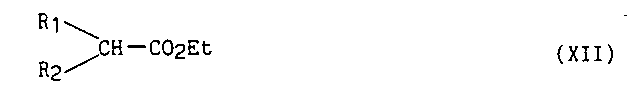 dans laquelle R₁ et R₂ ont la même signification que dans la formule (I),
   sur lequel on fait réagir le dérivé bromé de formule (III) défini précédemment,
   pour conduire au composé de formule (XIII) : 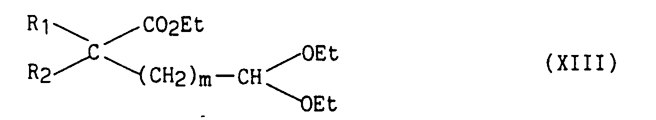 dans laquelle R₁ et R₂ ont la même signification que dans la formule (I), que l'on transforme :
   - a: en amine correspondante de formule (XIV), via l'azoture, puis l'isocyanate correspondants ou via l'acide et la benzyloxy-carbonylamine correspondants, 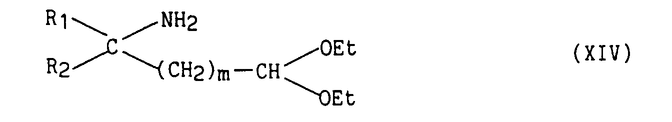 dans laquelle R₁ et R₂ ont la même signification que dans la formule (I),
   sur laquelle on réalise les mêmes réactions que celles décrites précédemment, pour le passage du composé (V) au composé (VII),
   pour conduire au composé de formule (XV) : 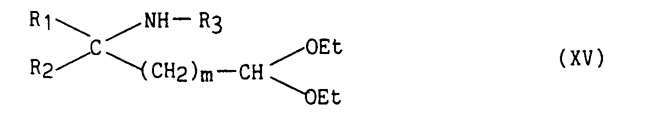 dans laquelle R₁, R₂ et m ont la même signification que dans la formule (I),
   ou bien, que l'on transforme en aldéhyde de formule (XVIa) en présence d'acide formique : 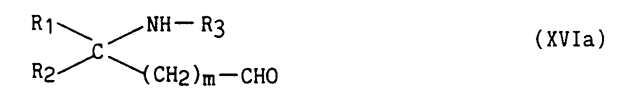 dans laquelle R₁ et R₂ ont la même signification que dans la formule (I),
   ou bien, lorsque m = 2, que l'on cyclise au reflux du toluène en composé de formule (XVIb), en présence d'acide p.toluène sulfonique : 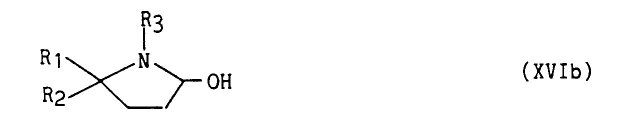 dans laquelle R₁, R₂ et R₃ ont la même signification que précédemment,
   composé de formule (XVIa) ou (XVIb) que l'on fait réagir avec l'ylure de formule (IX) décrit précédemment,
   pour conduire au composé de formule (I/c), cas particulier des composés de formule (I), 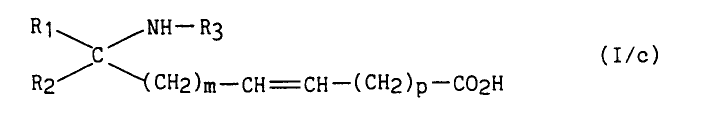 dans laquelle R₁, R₂, R₃ et m ont la même signification que dans la formule (I),
   - b: en alcool correspondant de formule (XVII) à l'aide d'un excès d'hydrure de lithium aluminium, 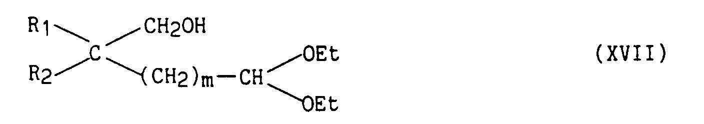 dans laquelle R₁, R₂ et m ont la même signification que dans la formule (I),
   puis, après passage par le mésylate, est transformé en nitrile correspondant de formule (XVIII) : 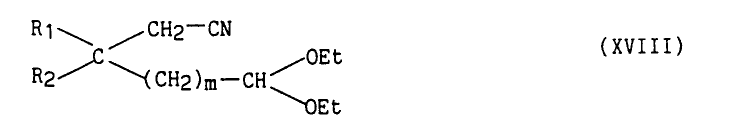 dans laquelle R₁, R₂ et m ont la même signification que dans la formule (I),
   qui est réduit en amine correspondant de formule (XIX) : 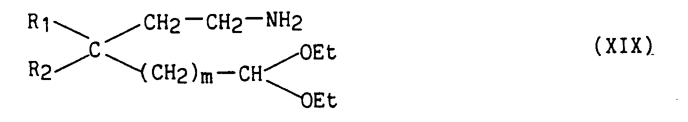 dans laquelle R₁, R₂ et m ont la même signification que dans la formule (I),
   sur laquelle on réalise les mêmes réactions que celles décrites précédemment, pour le passage du composé (V) au composé (VII),
   pour conduire au composé de formule (XX) : 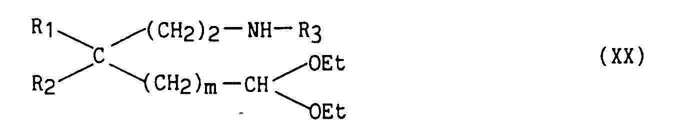 dans laquelle R₁, R₂, R₃ et m ont la même signification que dans la formule (I),
   qui, soit après déprotection à l'acide formique, soit cyclisation au reflux du toluène en présence d'acide p.toluène sulfonique (lorsque m représente 1 ou 2), puis réaction de l'ylure de formule (IX) décrit précédemment, est transformé en composé de formule (I/d), cas particulier des composés de formule (I), 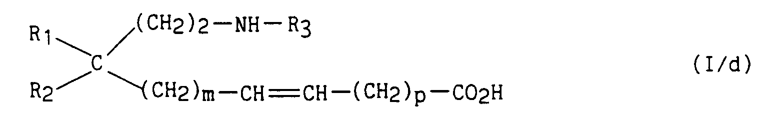 dans laquelle R₃, m et p ont la même signification que dans la formule (I),

   - composés de formule (I/a), (I/b), (I/c) ou (I/d) que l'on réduit, si on le souhaite, par hydrogénation catalytique pour conduire respectivement aux composés de formules (I/a₁), (I/b₁), (I/c₁) ou (I/d₁), cas particulier des composés de formule (I), 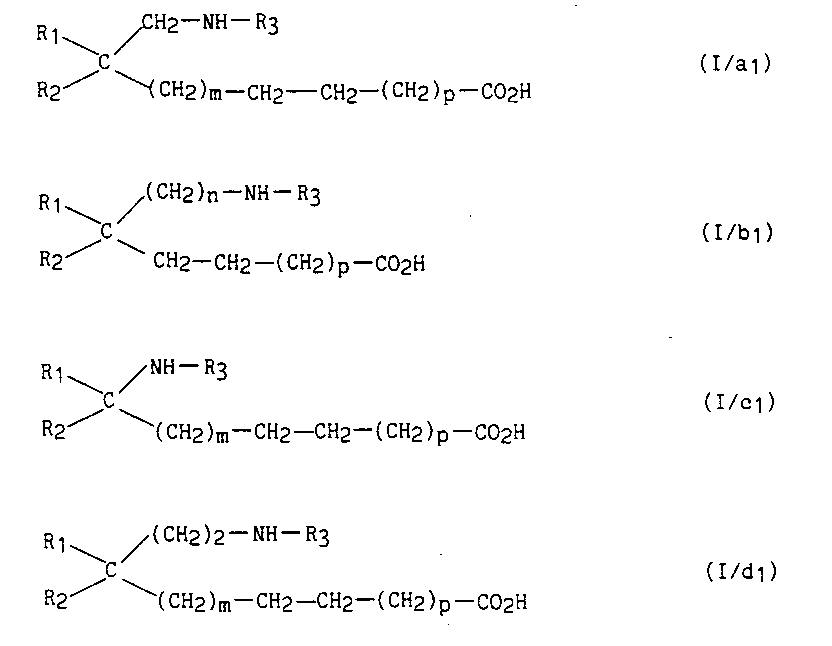 dans lesquelles R₁, R₂, m, n, p ont la même signification que dans la formule (I),
   - composés de formules (I/a), (I/a₁), (I/b), (I/b₁), (I/c), (I/c₁), (I/d) ou (I/d₁) :
      - que l'on purifie selon des techniques classiques de purification,
      - dont on sépare, éventuellement, les isomères, selon des techniques classiques de séparation,
      - et que l'on transforme, si on le souhaite, en leurs sels d'addition à un acide ou à une base pharmaceutiquement acceptable.

De plus, les composés répondant plus spécifiquement à la formule (I'), cas particulier des composés de formule (I) 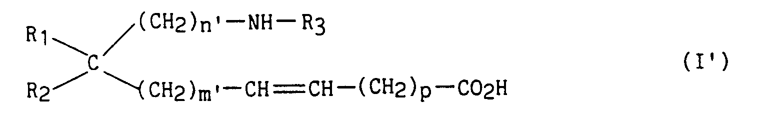 dans laquelle R₁, R₂, R₃ et p ont la même signification que dans la formule (I),
n' = 1, 2
m' = 1, 2
peuvent également être obtenus en utilisant le procédé caractérisé en ce que l'on utilise comme produit de départ un composé de formule (XXI) : 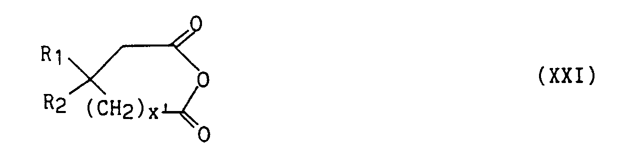 dans laquelle R₁ et R₂ ont la même signification que dans la formule (I') et x' est égal à 0 ou 1,
que l'on réduit en présence de borohydrure de sodium dans du méthanol pour conduire au composé de formule (XXII) : 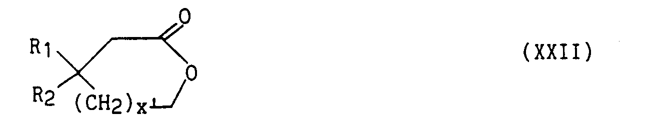 dans laquelle R₁, R₂ et x' ont la même signification que précédemment qui subit l'action :
- soit: de l'hydrure de diisobutylaluminium dans du toluène, pour conduire au composé de formule (XXIII) : 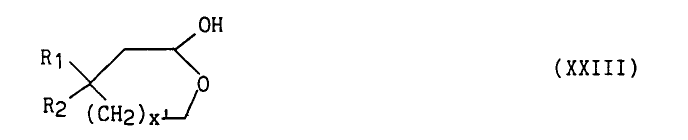 dans laquelle R₁, R₂ et x' ont la même signification que précédemment,
que l'on fait réagir avec l'ylure de phosphore de formule (IX) :

(C₆H₅)₃P=CH―(CH₂)ₚ―CO₂H (IX)

dans laquelle p a la même signification que dans la formule (I'),
pour conduire, après action du diazométhane, au composé de formule (XXIV) : 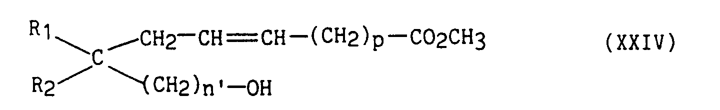 dans laquelle R₁, R₂, p et n' ont la même signification que dans la formule (I'),
que l'on fait réagir avec le chlorure de mésyle en présence de triéthylamine dans du toluène,
pour conduire au composé de formule (XXV) : 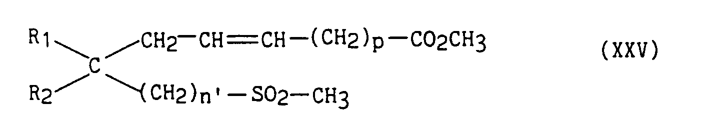 dans laquelle R₁, R₂, p et n' ont la même signification que dans la formule (I'),
que l'on fait réagir avec une solution de ditertbutyliminodicarbonate dans du diméthylformamide que l'on a fait préalablement réagir avec une suspension d'hydrure de sodium dans du diméthylformamide,
pour conduire au composé de formule (XXVI) : 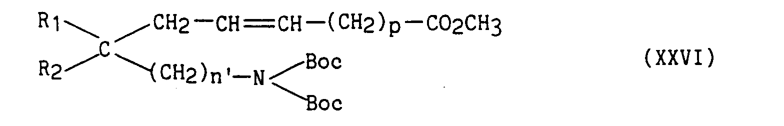 dans laquelle R₁, R₂, p et n' ont la même signification que dans (I'),
qui est transformé en amine correspondante de formule (XXVII) par réaction dans de l'éther chlorhydrique : 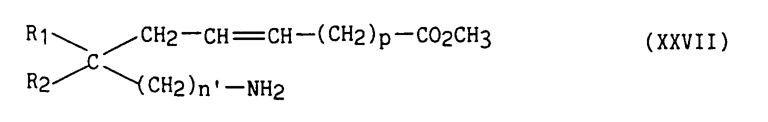 dans laquelle R₁, R₂, p et n' ont la même signification que dans la formule (I'),
que l'on transforme en composé de formule (XXVIII), 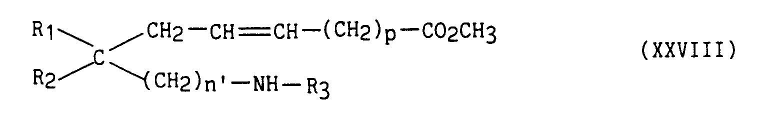 dans laquelle R₁, R₂, R₃, p et n' ont la même signification que dans la formule (I'),
par réaction, selon la nature de R₃
- soit avec un dérivé halogéné de formule (VI) en présence de triéthylamine dans du toluène :

   R'₃X (VI)

   dans laquelle X représente un atome d'halogène,
   R'₃ représente un radical phénylsulfonyl substitué ou non, ou un radical acyle,
- soit avec un isocyanate de formule R''-N=C=O (VI') dans laquelle R'' représente un radical alkyle et dans ce cas le radical R₃ de la formule (VII) est un radical alkylaminocarbonyl,
- soit avec un aldéhyde de formule R''₃ CHO (VI'') dans laquelle R''₃ est un radical alkyle pour former une imine qui est ensuite réduite en amine correspondante,
que l'on saponifie pour conduire au composé de formule (I/e), cas particulier des composés de formule (I'), 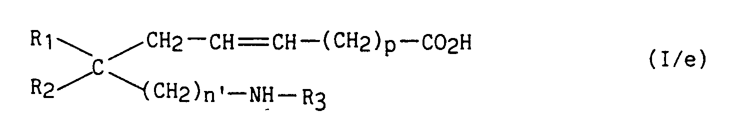 dans laquelle R₁, R₂, R₃, p et n' ont la même signification que dans la formule (I')
- soit: du cyanure de potassium dans du diméthylsulfoxyde pour conduire au composé de formule (XXIX) : 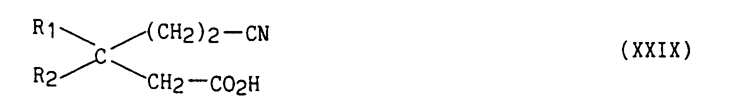 dans laquelle R₁ et R₂ ont la même signification que dans la formule (I'),
que l'on fait réagir avec du chloroformiate d'éthyle dans de l'acétone en présence de triéthylamine, pour conduire au composé de formule (XXX) : 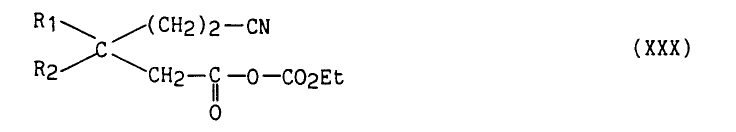 dans laquelle R₁ et R₂ ont la même signification que dans la formule (I'),
qui subit l'action de l'azoture de sodium en milieu aqueux, puis après chauffage dans du toluène l'action du tertbutanol,
pour conduire au composé de formule (XXXI) : 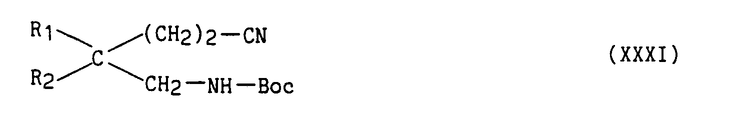 dans laquelle R₁ et R₂ ont la même signification que dans la formule (I'),
que l'on réduit en aldéhyde correspondant de formule (XXXII) en présence de trois équivalents d'hydrure de diisobutylaluminium dans du toluène : 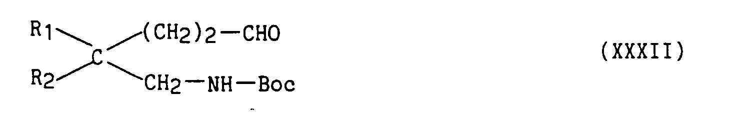 dans laquelle R₁ et R₂ ont la même signification que dans la formule (I'),
que l'on met en réaction avec l'ylure de phosphore de formule (IX) décrit précédemment,
pour conduire au composé de formule (XXXIII) : 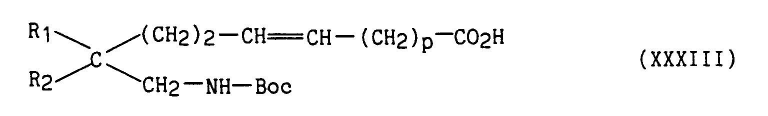 dans laquelle R₁, R₂ et p ont la même signification que dans la formule (I'),
dont la fonction amine est déprotégée dans de l'éther chlorhydrique, puis qui subit les mêmes réactions que celles décrites pour le passage du composé (XXVII) au composé (XXVIII),
pour conduire au composé de formule (I/f), cas particulier des composés de formule (I), 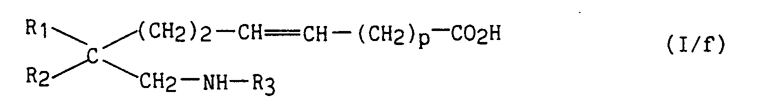 dans laquelle R₁, R₂, R₃ et p ont la même signification que dans la formule (I'),

- composés de formules (I/e) ou (I/f) que l'on réduit, si on le souhaite, par hydrogénation catalytique pour conduire respectivement aux composés de formule (I/e₁) ou (I/f₁), cas particulier des composés de formule (I) : 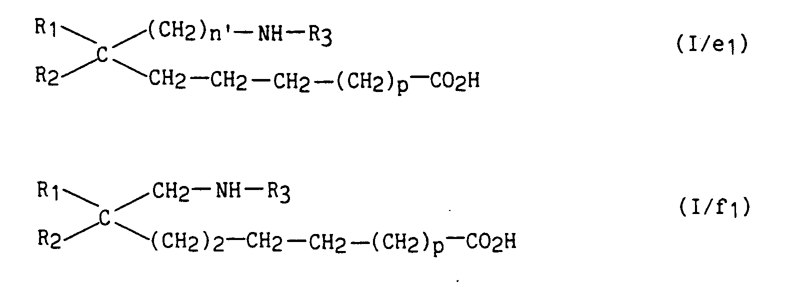 dans lesquelles R₁, R₂, p et n' ont la même signification que dans la formule (I),
- composés de formules (I/e), (I/e₁), (I/f) ou (I/f₁) :
   - que l'on purifie selon des techniques classiques de purification,
   - dont on sépare, éventuellement, les isomères, selon des techniques classiques de séparation,
   - et que l'on transforme, si on le souhaite, en leurs sels d'addition à un acide ou à une base pharmaceutiquement acceptable.

Les composés de formule (I) possèdent des propriétés pharmacologiques très intéressantes. En particulier, ils sont capables d'inhiber l'agrégation plaquétaire induite par le U46619 (9,11-didéoxy-11α,9α-époxyméthanoprostaglandine F_{2α}), agoniste des récepteurs TXA₂, d'inhiber les contractions provoquées par le U46619 sur la trachée de cobaye et de prévenir in vivo les bronchoconstrictions induites par le U46619 chez le cobaye. En plus, les composés inhibent la synthèse de TXA₂ dans le sang du lapin.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 10 et 200 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

### Exemple 1: Acide (5Z)-10-[(4-chlorophényl)sulfonamido]-8,8- pentaméthylène dèc-5-ènoïque, sel de sodium

### Stade A : 4,4-Pentaméthylènylvalérolactone

A 372 mmoles de borohydrure de sodium en suspension dans 74 ml de tétrahydrofurane (THF), sont ajoutés, goutte à goutte, à 0°C, 372 mmoles d'anhydride de l'acide 1,1-cyclohexane-diacétique (préparé à partir du diacide correspondant) dissoutes dans 300 ml de THF. Après retour à température ambiante, l'agitation est maintenue une heure. L'ensemble est à nouveau refroidi à 0°C, puis 216 ml d'éthanol chlorhydrique 6,9 N sont ajoutés à ce mélange. L'ensemble est alors porté une heure à reflux.
Après filtration et évaporation, le produit attendu est obtenu sous forme d'une huile qui est distillée sous vide.
Rendement : 62 %
Point d'ébullition : 166-175°C/20 mmHg

### Stade B : 4,4-Pentaméthylènylvalérolactol

Après avoir placé sous atmosphère inerte 65,4 mmoles du produit obtenu au stade A dans 200 ml de toluène refroidi à -80°C, sont ajoutés 87,2 ml d'une solution 1,5 M d'hydrure de diisobutylaluminium dans du toluène. Lorsque la lactone a disparu, 50 ml d'acétate d'éthyle sont ajoutés au mélange précédent puis 50 ml d'eau. L'ensemble est laissé une heure sous agitation à température ambiante. Après filtration et évaporation du solvant, le produit attendu est obtenu sous forme d'une huile jaune.
Rendement : 90 %

### Stade C : (5Z)-10-Hydroxy-8,8-pentaméthylène dèc-5-ènoate de méthyle

Après avoir placé sous atmosphère inerte 82,2 mmoles du bromure de l'acide 5-triphénylphosphoniopentanoïque dans 60 ml de THF, sont ajoutés 164,5 ml d'une solution 1M de tertbutanolate de potassium dans le THF. L'ensemble est laissé une heure sous agitation à température ambiante. Il est alors refroidi à 0°C et 41,1 mmoles du produit obtenu au stade B dissoutes dans 80 ml de THF sont ajoutées goutte à goutte. Après l'addition, l'ensemble est maintenu sous agitation à température ambiante jusqu'à disparition complète du lactol. Après concentration, la phase aqueuse est extraite par 100 ml de dichlorométhane, puis acidifiée par de l'acide chlorhydrique concentré jusqu'à pH = 1. Le milieu est alors extrait par du dichlorométhane. Les phases organiques sont réunies, séchées et évaporées et conduisent à un résidu qui est repris par de l'éther, puis filtré. Le filtrat évaporé conduit à 10,6 g d'une huile jaune qui est dissoute dans 300 ml d'éther. On ajoute alors, à 0°C, une solution éthérée de diazométhane à -40°C jusqu'à persistance d'une coloration jaune. Le milieu réactionnel est abandonné une heure à 0°C. Après addition de quelques gouttes d'acide acétique pour détruire l'excès de diazométhane, l'ensemble est concentré sous vide. Le produit attendu est purifié par chromatographie sur colonne de silice en utilisant comme éluant un mélange toluène/éthanol (95/5).
Rendement : 68 %
Résonance Magnétique Nucléaire du Proton (Pyridine d₅/TMS) :
Constante de couplage entre les protons éthvléniaues cis : J = 11,6 Hz

### Stade D : (5Z)-10-Méthylsulfonyloxy-8,8-pentaméthylène dèc-5-ènoate de méthyle

28 mmoles du composé obtenu au stade C et 36,6 mmoles de triéthylamine sont placées dans 100 ml d'éther. On ajoute alors, goutte à goutte, à température ambiante 33,5 mmoles du chlorure de l'acide méthanesulfonique en solution dans 50 ml d'éther. Le produit attendu est alors obtenu après évaporation du solvant, sous forme d'une huile jaune.
Rendement : 81 %

### Stade E : (5Z)-10-[Bis(tertbutoxycarbonyl)amino]-8,8-pentaméthylène dèc-5-ènoate de méthyle

Sous atmosphère inerte, à température ambiante, 50 ml d'une solution contenant 30 mmoles de ditertbutyliminodicarbonate dans du diméthylformamide (DMF) anhydre sont ajoutés à une suspension contenant 30 mmoles d'hydrure de sodium dans 30 ml de DMF anhydre. L'ensemble est chauffé 5 heures, à 60°C, sous agitation. 22,5 mmoles du composé obtenu au stade D dissoutes dans 100 ml de DMF anhydre sont ajoutées et le chauffage est maintenu 4 heures. Le milieu est acidifié par 500 ml d'acide chlorhydrique 2N puis extrait par du dichlorométhane. Après séchage et évaporation des solvants, le produit attendu est obtenu sous forme d'une huile jaune.
Rendement : 98 %

### Stade F : (5Z)-10-Amino-8,8-pentaméthylène dèc-5-ènoate de méthyle, chlorhydrate

A une solution contenant 22,2 mmoles du produit obtenu au stade E dans 30 ml d'éther, sont ajoutés, goutte à goutte, 200 ml d'éther chlorhydrique 3,3 N à température ambiante. L'agitation est maintenue 15 heures. Après évaporation des solvants, le résidu est repris par de l'éther. Le produit attendu est obtenu après filtration et évaporation sous forme d'une huile jaune.
Rendement : 96 %

### Stade G : (5Z-10-[(4-chlorophényl)sulfonamido]-8,8-pentaméthylène dèc-5-ènoate de méthyle

4,5 mmoles du chlorure de l'acide 4-chlorophénylsulfonique dissoutes dans 30 ml d'éther sont ajoutées, goutte à goutte, à température ambiante à une solution contenant 5,4 mmoles de triéthylamine et 4,5 mmoles du composé obtenu au stade F dans 50 ml d'éther. A la fin de la réaction, les sels sont filtrés et le filtrat est évaporé. Le produit attendu est obtenu après purification du résidu par chromatographie sur colonne de silice en utilisant comme éluant un mélange dichlorométhane/acétone (95/5).
Rendement : 53 %

### Stade H : Acide (5Z)-10-[(4-chlorophényl)sulfonamido]-8,8-pentaméthylène dèc-5-ènoïque, sel de sodium

770 mmoles du produit obtenu au stade G sont dissoutes dans 3 ml de méthanol en présence de 3 ml de soude 1N. La solution est concentrée sous vide et le résidu dilué par 20 ml d'eau. La phase aqueuse est extraite par de l'éther puis acidifiée par une solution d'acide chlorhydrique 1N (pH - 1). Après extraction au dichlorométhane, les phases organiques sont séchées, puis évaporées. Le résidu est dilué par 5 ml de méthanol. On ajoute alors 7,7 ml de soude N/10. L'ensemble est agité une heure à température ambiante puis évaporé. Le produit attendu cristallise dans de l'éther.
Rendement : 100 %
Point de fusion : 145-147°C
Résonance Magnétique Nucléaire du Proton (Pyridine d₅/TMS)
Constante de couplage entre les protons éthylèniques cis : J = 11,6 Hz

| Microanalyse élémentaire : | | | | | |
|---|---|---|---|---|---|
| | C % | H % | N % | Cl % | S % |
| calculé | 56,05 | 6,50 | 3,11 | 7,88 | 7,13 |
| trouvé | 55,77 | 6,83 | 3,31 | 8,15 | 6,79 |

Les exemples 2 à 5 ont été obtenus selon le même mode opératoire que celui décrit pour l'exemple 1 en utilisant les produits de départ correspondants.

### Exemple 2 : Acide (4Z)-9-[(4-chlorophényl)sulfonamido]-7,7-pentaméthyléne non-4-ènoïque, sel de sodium

Point de fusion : 135-138°C
Résonance Magnétique Nucléaire du Proton (Pyridine d₅/TMS)
Constante de couplage entre les protons éthylèniques cis : J = 10,3 Hz

| Microanalyse élémentaire : | | | | | |
|---|---|---|---|---|---|
| | C % | H % | N % | Cl % | S % |
| calculé | 55,10 | 6,24 | 3,21 | 8,13 | 7,35 |
| trouvé | 55,24 | 6,69 | 3,24 | 8,48 | 7,05 |

### Exemple 3 : Acide (4Z)-9-[(4-chlorophényl)sulfonamido]-7,7-tétraméthylène non-4-ènoïque, sel de sodium

Point de fusion : 180°C

| Microanalyse élémentaire : | | | | | |
|---|---|---|---|---|---|
| | C % | H % | N % | Cl % | S % |
| calculé | 54,09 | 5,97 | 3,32 | 8,40 | 7,60 |
| trouvé | 54,02 | 6,22 | 3,42 | 8,25 | 7,51 |

### Exemple 4: Acide (5Z)-10-[(4-méthylphényl)sulfonamido]-8,8-pentaméthylène dèc-5-ènoïque, sel de sodium

Point de fusion : 79-85°C

| Microanalyse élémentaire : | | | | |
|---|---|---|---|---|
| | C % | H % | N % | S % |
| calculé | 61,52 | 7,51 | 3,26 | 7,46 |
| trouvé | 61,33 | 7,79 | 3,35 | 7,39 |

### Exemple 5 : Acide (5Z)-10-[(4-Bromophényl)sulfonamido]-8,8-pentaméthylène dèc-5-ènoïque, sel de sodium

Point de fusion : 130-133°C

| Microanalyse élémentaire : | | | | | |
|---|---|---|---|---|---|
| | C % | H % | N % | Br | S % |
| calculé | 51,02 | 5,91 | 2,83 | 16,16 | 6,49 |
| trouvé | 50,76 | 6,13 | 2,96 | 16,29 | 6,64 |

### Exemple 6 : Acide (4Z)-8-[(4-chlorophényl)sulfonamido]-7,7-tétraméthylène oct-4-ènoïque, sel de sodium

### Stade A : 1-(2,2-Diméthoxyéthyl)cyclopentanecarbonitrile

A une solution agitée à 78°C sous atmosphère d'argon de 0,1 mole de diisopropylamidure de lithium (préparée à partir de 0,1 mole de diisopropylamine et de 63 ml de butyllithium 1,6 M dans l'hexane) dans 250 ml de THF anhydre, on ajoute par un goutte à goutte rapide, 0,1 mole de cyclopentanecarbonitrile dans 50 ml de THF. On agite 5 minutes à cette température avant d'ajouter 0,11 mole de 1-bromo-2,2-diméthoxyéthane. On laisse le milieu revenir à température ambiante et on hydrolyse à l'aide de 150 ml d'eau. Le milieu est décanté, la phase aqueuse extraite 2 fois avec 150 ml d'éther. Les phases organiques réunies sont lavées avec 100 ml d'eau salée, 100 ml d'eau puis séchées sur sulfate de magnésium. Les solvants sont évaporés sous vide et l'huile résiduelle est distillée.
Point d'ébullition : 84°C (p = 0,2 mmHg)
Rendement : 54 %

### Stade B : 1-(2,2-Diméthoxyéthyl)cyclopentaneméthylamine

A 109 mmoles d'hydrure de lithium et d'aluminium en suspension dans 200 ml d'éther agités à température ambiante sous atmosphère d'azote, sont ajoutés goutte à goutte 54 mmoles du produit obtenu au stade A dans 100 ml d'éther. Lorsque tout le nitrile a disparu, 10 ml d'acétate d'éthyle sont ajoutés au mélange puis 10 ml d'eau. L'ensemble est agité 1 heure, filtré, séché sur Mg SO₄ et le solvant est évaporé. Le produit attendu est obtenu avec un rendement de 88 %.

### Stade C : N-(4-Chlorophényl)sulfonyl-1-(2,2-diméthoxyéthyl)cyclopentaneméthylamine

Obtenu selon le même mode opératoire que celui décrit dans le stade G de l'exemple 1 en utilisant comme produit de départ le produit obtenu au stade précédent.
Rendement : 96 %

### Stade D : N-[(4-Chlorophényl)sulfonyl]-3-hydroxy-2-azaspiro[4.4]nonane

Une solution de 31,7 mmoles du produit obtenu au stade précédent et de 20,1 mmoles d'acide paratoluènesulfonique dans 280 ml de THF et 60 ml d'eau est portée au reflux 8 heures. Après retour à la température ambiante, le milieu réactionnel est concentré aux 4/5 de son volume. Le produit est extrait par 200 ml de CH₂Cl₂, la phase organique est lavée avec 50 ml d'une solution saturée de bicarbonate de sodium, 50 ml d'eau salée et 50 ml d'eau. Après séchage de la phase organique sur MgSO₄, le solvant est évaporé sous vide.
Rendement : 63 %

### Stade E : Acide (4Z)-8-[(4-chlorophényl)sulfonamido]-7,7-tétraméthylène oct-4-ènoïque

Après avoir placé sous atmosphère inerte 40 mmoles du chlorure de l'acide 4-triphénylphosphoniobutanoïque dans 60 ml de THF, sont ajoutés 80 ml d'une solution 1M de tertbutanolate de potassium dans le THF. L'ensemble est laissé 1 heure sous agitation à température ambiante. Il est alors refroidi à 0°C et 20 mmoles du produit obtenu au stade précédent dissoutes dans 40 ml de THF sont ajoutées goutte à goutte. Après addition, l'ensemble est maintenu sous agitation à température ambiante jusqu'à disparition complète du lactame. Après concentration, la phase aqueuse est extraite par 100 ml de CH₂Cl₂, puis acidifiée par de l'acide chlorhydrique concentré jusqu'à pH = 1. Le milieu est alors extrait par du CH₂Cl₂. Les phases organiques sont réunies, séchées et évaporées. Le résidu huileux est purifié par chromatographie sur colonne de silice en utilisant comme éluant un mélange CH₂Cl₂/méthanol/acide acétique (95/4/1). Le produit attendu cristallise.
Rendement : 52 %
Point de fusion : 100°C

### Stade F : Acide (4Z)-8-[(4-chlorophényl)sulfonamido]-7,7-tétraméthylène oct-4-ènoïque, sel de sodium

Le produit obtenu au stade précédent est dilué par 10 ml de méthanol. On ajoute alors 10,4 ml de soude N. L'ensemble est agité 1 heure à température ambiante puis évaporé. Le produit attendu cristallise dans l'éther.
Rendement : 100 %
Résonance Mannétique Nucléaire du Proton (DMSO d6) :
Constante de couplage entre les protons éthyléniques cis : J = 11,5 Hz

| Microanalyse élémentaire : | | | | | |
|---|---|---|---|---|---|
| | C % | H % | N % | Cl % | S % |
| calculé | 53,00 | 5,68 | 3,43 | 8,69 | 7,86 |
| trouvé | 53,40 | 5,73 | 3,55 | 8,72 | 7,63 |

### Exemple 7: Acide (4Z)-8-[(4-chlorophényl)sulfonamido]-7,7-pentaméthylène oct-4-ènoïque, sel de sodium

Préparé selon le même mode opératoire que celui décrit pour l'exemple 6.
Point de fusion : 194°C
Rendement : 80 %
Résonance Magnétique Nucléaire du Proton (DMSO d6) :
Constante de couplage entre les protons éthyléniques cis : J = 11,8 Hz

| Microanalyse élémentaire : | | | | | |
|---|---|---|---|---|---|
| | C % | H % | N % | Cl % | S % |
| calculé | 54,09 | 5,97 | 3,32 3,56 | 8,40 | 7,60 |
| trouvé | 54,68 | 6,18 | | 8,67 | 7,44 |

### Exemple 8: Acide (5Z)-9-[(4-chlorophényl)sulfonamido]-8,8-tétraméthylène non-5-ènoïque, sel de sodium

Préparé selon le mode opératoire décrit pour l'exemple 6.
Rendement : 66 %
Point de fusion : 124°C
Résonance Magnétique Nucléaire du Proton (CDCl₃) :
Constante de couplage entre les protons éthyléniques cis : J = 11,6 Hz

| Microanalyse élémentaire : | | | | | |
|---|---|---|---|---|---|
| | C % | H % | N % | Cl % | S % |
| calculé | 54,09 | 5,97 | 3,32 | 8,40 | 7,60 |
| trouvé | 53,78 | 6,05 | 3,61 | 8,55 | 7,46 |

### Exemple 9: Acide (4Z)-9-[(4-chlorophényl)sulfonamido]-8,8-tétraméthylène non-4-ènoïque, sel de sodium

Préparé selon le mode opératoire décrit dans l'exemple 6.
Rendement : 63 %
Résonance Magnétique Nucléaire du Proton (DMSO d6) :
Constante de couplage entre les protons éthyléniques cis : J = 11,6 Hz

| Microanalyse élémentaire : | | | | | |
|---|---|---|---|---|---|
| | C % | H % | N % | Cl % | S % |
| calculé | 54,09 | 5,97 | 3,32 | 8,40 | 7,60 |
| trouvé | 54,27 | 6,41 | 3,46 | 8,39 | 7,10 |

### Exemple 10 : Acide (4Z)-8-[(4-chlorophényl)sulfonamido]-8,8-tétraméthylène oct-4-ènoïque, sel de sodium

### Stade A : 1-(3,3-Diméthoxypropyl)cyclopentanecarboxylate de méthyle

Préparé selon un mode opératoire identique au stade A de l'exemple 6 à partir du cyclopentanecarboxylate de méthyle et de l'acétaldiméthylique du 3-bromopropionaldéhyde avec un rendement de 72 %.

### Stade B : Acide 1-(3,3-diméthoxypropyl)cyclopentanecarboxylique

94 mmoles du produit préparé au stade précédent sont dilués dans 100 ml de méthanol. Après avoir ajouté 25 ml de soude à 35 %, le milieu est chauffé au reflux 2 heures, puis, après retour à l'ambiante, extrait par 200 ml de CH₂Cl₂. La phase aqueuse est acidifiée jusqu'à pH = 5 par HClN et extraite 3 fois à l'aide de 100 ml de CH₂Cl₂. Les phases organiques réunies, séchées sur sulfate de magnésium sont évaporées sous vide.
Rendement : 88 %

### Stade C : N-Benzyloxycarbonyl-1-(3,3-diméthoxypropyl)cyclopentylamine

Une solution contenant 23,1 mmoles du produit préparé au stade précédent, 25,4 mmoles de diphénylphosphorylazide (DPPA) et de 27,7 mmoles de triéthylamine dans 100 ml de benzène anhydre est portée au reflux pendant 3 heures. Après concentration du solvant, le résidu est chromatographié sur silice en employant comme éluant le mélange CH₂Cl₂/acétone (95/5). On récupère ainsi 21,9 mmoles (95 %) d'une huile qui possède en IR une bande d'absorption à 2256 cm⁻¹. Cette huile est diluée par 25 ml de DMF anhydre. On ajoute 21,9 mmoles d'alcool benzylique et 21,9 mmoles de CuCl et on agite le milieu réactionnel 45 minutes à température ambiante. Le milieu est alors dilué par 100 ml d'éther et lavé avec 50 ml d'eau et 25 ml d'eau salée puis séchée sur sulfate de magnésium. Après concentration des solvants, le produit est purifié par chromatographie sur silice en utilisant comme éluant le mélange CH₂Cl₂/acétone (95/5).
Rendement : 73 %

### Stade D : 1-(3,3-Diméthoxypropyl)cyclopentylamine

Le produit obtenu dans l'étape précédente, dilué dans 50 ml de méthanol est aigité à température ambiante sous atmosphère d'hydrogène en présence de 100 mg de palladium sur charbon à 10 %. Le milieu est filtré, le solvant concentré. Le produit attendu est une huile incolore.
Rendement : 100 %

### Stade E : N-(4-Chlorophényl)sulfonyl-1-(3,3-diméthoxypropyl) cyclopentylamine

Préparé selon le mode opératoire décrit dans le stade G de l'exemple 1.
Rendement : 83 %

### Stade F : N-[(4-Chlorophényl)sulfonyl]-2-hydroxy-1-azaspiro[4.4]nonane

Préparé selon le mode opératoire décrit dans le stade D de l'exemple 6.
Rendement : 70 %

### Stade G : Acide (4Z)-8-[(4-chlorophényl)sulfonamido]-8,8-tétraméthylène oct-4-énoïque

Préparé selon le mode opératoire décrit au stade E de l'exemple 6.
Rendement : 68 %

### Stade H : Acide (4Z)-8-[(4-chlorophényl)sulfonamido]-8,8-tétraméthylène oct-4-énoïque, sel de sodium

Préparé selon le mode opératoire décrit dans le stade F de l'exemple 6.
Rendement : 100 %
Point de fusion : 99,5°C

| Microanalyse élémentaire : | | | | | |
|---|---|---|---|---|---|
| | C % | H % | N % | Cl % | S % |
| calculé | 53,00 | 5,68 | 3,43 | 8,69 | 7,86 |
| trouvé | 52,91 | 5,78 | 3,74 | 8,82 | 7,98 |

### Exemple 11 : Acide (5Z)-9-[(4-chlorophényl)sulfonamido]-9,9-tétraméthylène non-5-ènoïque, sel de sodium

Obtenu selon le même mode opératoire que celui décrit pour l'exemple 10 en utilisant les produits de départ correspondants.
Point de fusion : 163°C
Résonance Magnétique Nucléaire du Proton (DMSO d6) :
Constante de couplage entre les protons éthyléniques cis : J = 10,8 Hz

| Microanalyse élémentaire : | | | | | |
|---|---|---|---|---|---|
| | C % | H % | N % | Cl % | S % |
| calculé | 54,09 | 5,97 | 3,32 | 8,40 | 7,60 |
| trouvé | 53,59 | 5,95 | 3,60 | 8,65 | 7,36 |

### Exemple 12 : Acide (5Z)-10-[(4-chlorophényl)sulfonamido]-8,8-tétraméthylène dèc-5-ènoïque, sel de sodium

Préparé selon le même mode opératoire que celui décrit pour l'exemple 1 en utilisant les produits de départ correspondants.
Point de fusion : 117°C
Résonance Magnétique Nucléaire du Proton (DMSO d6) :
Constante de couplage entre les protons éthyléniques cis : J = 10,8 Hz

| Microanalyse élémentaire : | | | | | |
|---|---|---|---|---|---|
| | C % | H % | N % | Cl % | S % |
| calculé | 55,10 | 6,24 | 3,21 | 8,13 | 7,35 |
| trouvé | 55,67 | 6,70 | 3,22 | 7,86 | 7,55 |

### Exemple 13: Acide (4Z)-9-[(1-naphtyl)sulfonamido)-7,7-tétraméthylène non-4-ènoïque, sel de sodium

Préparé selon le mode opératoire décrit pour l'exemple 1 en utilisant les produits de départ correspondants.
Point de fusion : 141-142°C

| Microanalyse élémentaire : | | | | |
|---|---|---|---|---|
| | C % | H % | N % | S % |
| calculé | 63,14 | 6,45 | 3,20 | 7,33 |
| trouvé | 62,82 | 6,64 | 3,38 | 7,48 |

### Exemple 14 : Acide (4Z)-10-[(4-chlorophényl)sulfonamido]-8,8-tétraméthylène dèc-4-ènoïque, sel de sodium

### Stade A : 1-(3,3-Diméthoxypropyl)cyclopentaneméthanol

A une suspension agitée à 0°C de 0,25 mole d'hydrure de lithium et d'aluminium dans 200 ml d'éther, on ajoute goutte à goutte une solution de 0,25 mole du composé préparé au stade A de l'exemple 10 dans 200 ml d'éther. L'addition terminée, on laisse le milieu réactionnel remonter à l'ambiante puis on agite 4 heures à cette température avant d'hydrolyser par addition de 20 ml d'eau. Les sels sont filtrés, lavés deux fois à l'éther, le filtrat est séché et concentré.
Rendement : 77 %

### Stade B : Acétaldiméthylique du 1-méthylsulfonyloxyméthyl cyclopentane propionaldéhyde

Préparé à partir du composé précédent selon le même mode opératoire que celui décrit au stade D de l'exemple 1.
Rendement : 95 %

### Stade C : 1-(3,3-Diméthoxypropyl)cyclopentanacétonitrile

Une solution contenant 15,4 mmoles du produit préparé au stade précédent et de 16,9 mmoles de cyanure de potassium dans 300 ml de DMSO est chauffée 15 heures à 100°C. Le milieu refroidi est hydrolysé sur 600 ml d'eau glacée et est extrait par 3 fois 150 ml d'acétate d'éthyle. Les phases organiques réunies sont séchées sur sulfate de magnésium et après évaporation des solvants, le produit attendu est purifié par chromatographie sur silice en utilisant comme éluant le mélange toluène/acétone (95/5).
Rendement : 60 %

### Stade D : 1-(3,3-Diméthoxypropyl)cyclopropanéthylamine

Préparé selon le mode opératoire décrit au stade B de l'exemple 6.
Rendement : 90 %

### Stade E : N-(4-chlorophényl)sulfonyl-1-(3,3-diméthoxypropyl) cyclopentanéthylamine

Préparé selon le mode opératoire décrit au stade G de l'exemple 1.
Rendement : 89 %

### Stade F : N-(4-Chlorophényl)sulfonyl-7-aza-8-hydroxyspiro[4.6]undécane

Préparé selon un mode opératoire identique au stade D de l'exemple 6.
Rendement : 80 %

### Stade G : Acide (4Z)-10-[(4-chlorophényl)sulfonamido]-8,8-tétraméthylène dèc-4-ènoïque

Préparé selon le mode opératoire décrit au stade E de l'exemple 6.
Rendement : 78 %

### Stade H : Acide (4Z)-10-[(4-chlorophényl)sulfonamido]-8,8-tétraméthylène dèc-4-ènoïque, sel de sodium

Préparé selon le mode opératoire décrit au stade F de l'exemple 6.
Rendement : 91 %
Point de fusion : 105°C
Résonance Magnétique Nucléaire du Proton (DMSO d6) :
Constante de couplage entre les protons éthyléniques cis : J = 11,1 Hz

| Microanalyse élémentaire : | | | | | |
|---|---|---|---|---|---|
| | C % | H % | N % | Cl % | S % |
| calculé | 55,10 | 6,24 | 3,21 | 8,13 | 7,35 |
| trouvé | 55,40 | 6,51 | 3,29 | 7,81 | 7,18 |

### Exemple 15 : Acide (4Z)-6-[1-{2-(4-chlorophényl)sulfonamidoéthyl}-4-tertbutylcyclohex-1-yl]hex-4-ènoïque, sel de sodium

### Stade A : Ester éthylique de l'acide (4-tert-butylcyclohexylidène) cyanoacétique

Dans un ballon surmonté d'un Deanstark, porter au reflux 4 heures un mélange de 0,13 mole de 4-tertbutylcyclohexanone, 0,195 mole de cyanoacétate d'éthyle, de 6 ml d'acide acétique, de 5 g d'acétate d'ammonium et de 200 ml de benzène. Après retour à température ambiante, le milieu réactionnel est concentré sous vide et le résidu est purifié par chromatographie sur silice en utilisant comme éluant le mélange CH₂Cl₂/hexane (70/30).
Rendement : 90 %
Point de fusion : 43°C

### Stade B : 9-Tertbutyl-1,5-dicyano-2,4-dioxo-3-azaspiro[5.5]undécane

A une solution agitée à température ambiante de 0,116 mole d'éthanolate de sodium dans 200 ml d'éthanol, couler goutte à goutte une solution de 0,116 mole de cyanoacétamide dans 200 ml d'éthanol. Agiter la suspension 1 heure à température ambiante avant d'ajouter par petites fractions 0,116 mole du composé obtenu au stade précédent. La solution résultante est agitée 12 heures à température ambiante avant d'être hydrolysée à l'aide de 300 ml d'eau. Le milieu est ensuite acidifié à pH = 1 par addition lente de la quantité nécessaire d'acide chlorhydrique 3N. Le précipité est filtré et lavé par un minimum d'alcool puis d'éther diisopropylique.
Rendement : 95 %
Point de fusion : 248°C

### Stade C : Acide 4-tertbutyl-1,1-cyclohexane diacétique

On porte au reflux pendant 5 jours une suspension de 0,11 mole du composé préparé au stade précédent dans 350 ml d'eau, 350 ml d'acide chlorhydrique concentré et 1050 ml d'acide acétique glacial. Le milieu est concentré sous vide, le résidu, repris dans 500 ml d'un mélange éther/éthanol (50/50), est filtré, le filtrat concentré et le résidu solide repris dans 150 ml de pentane.
Rendement : 85 %
Point de fusion : 178°C

### Stade D : 9-Tertbutyl-3-oxaspiro[5.5]undécane-2,4-dione

Une solution de 0,09 mole du composé préparé au stade précédent dans 300 ml d'anhydride acétique est porté au reflux pendant 12 heures. Après concentration sous vide, le résidu cristallisé est repris 2 fois dans 200 ml de pentane.
Rendement : 76 %
Point de fusion :110-112°C

### Stade E : 9-Tertbutyl-2-oxo-3-oxaspiro[5.5]undécane, mélange d'isomères

Préparé selon le mode opératoire du stade A de l'exemple 1, l'huile brute récupérée est cristallisée dans le pentane pour fournir après filtration l'isomère 1.
Rendement : 52 %
Point de fusion : 130°C

Le filtrat est concentré pour fournir l'isomère 2.
Rendement : 28 %
Point de fusion : 54°C

### Stade F : 9-Tertbutyl-2-hydroxy-3-oxaspiro[5.5]undécane, isomère 1

Préparé à partir de l'isomère 1 préparé précédemment selon un mode opératoire identique à celui utilisé au stade B de l'exemple 1.
Rendement : 59 %
Point de fusion : 143°C

### Stade G : (4Z)-6-[4-Tertbutyl-1-(2-hydroxyéthyl)cyclohex-1-yl]hex-4-ènoate de méthyle, isomère 1

Préparé selon un mode opératoire identique à celui du stade C de l'exemple 1.
Rendement : 90 %
Résonance Magnétique Nucléaire du Proton (CDCl₃) :
Constante de couplage entre les protons éthyléniques cis : J = 10,8 Hz

### Stade H : (4Z)-6-[4-Tertbutyl-1-(2-méthylsulfonyloxyéthyl)cyclohex-1-yl]hex-4-ènoate de méthyle, isomère 1

Préparé selon un mode opératoire identique à celui du stade D de l'exemple 1.
Rendement : 94 %

### Stade I : (4Z)-6-[4-Tertbutyl-1-[2-bis(tertbutoxycarbonyl)aminoéthyl] cyclohex-1-yl]hex-4-ènoate de méthyle, isomère 1

Préparé selon le même mode opératoire que celui utilisé au stade E de l'exemple 1.
Rendement : 61 %

### Stade J : (4Z)-6-[4-Tertbutyl-1-(2-aminoéthyl)cyclohex-1-yl]hex-4-ènoate de méthyle, chlorhydrate, isomère 1

Préparé selon un mode opératoire identique à celui utilisé au stade F de l'exemple 1.
Rendement : 100 %

### Stade K : (4Z)-6-[4-Tertbutyl-1-[N-(2-chlorophényl)sulfonamidoéthyl] cyclohex-l-yl]hex-4-ènoate de méthyle, isomère 1

Préparé selon un mode opératoire identique à celui utilisé au stade G de l'exemple 1.
Rendement : 78 %

### Stade L : Acide (4Z)-6-[4-tertbutyl-1-[N-(2-chlorophényl)sulfonamidoéthyl] cyclohex-1-yl]hex-4-ènoïque, sel de sodium, isomère 1

Préparé selon un mode opératoire identique à celui utilisé au stade H de l'exemple 1.
Rendement : 58 %
Point de fusion : 191°C

| Microanalyse élémentaire : | | | | | |
|---|---|---|---|---|---|
| | C % | H % | N % | Cl % | S % |
| calculé | 58,58 | 7,17 | 2,85 | 7,21 | 6,52 |
| trouvé | 58,15 | 7,27 | 3,04 | 7,26 | 6,89 |

### Exemple 16 : Acide (4Z)-6-[1-[2-(4-chlorophényl)sulfonamidoéthyl]indan-1-yl]hex-4-ènoïque, sel de sodium

Préparé selon le même mode opératoire que celui décrit dans l'exemple 15 en utilisant les produits de départ correspondants.
Rendement : 98 %
Point de fusion : 75-80°C
Résonance Magnétique Nucléaire du Proton (DMSO d6) :
Constante de couplage entre les protons éthyléniques cis : J = 10,8 Hz

| Microanalyse élémentaire : | | | | | |
|---|---|---|---|---|---|
| | C % | H % | N % | Cl % | S % |
| calculé | 58,78 | 5,36 | 2,98 | 7,54 | 6,82 |
| trouvé | 58,67 | 5,68 | 2,90 | 7,50 | 6,54 |

### Exemple 17 : Acide (5Z)-7-[1-[2-(4-chlorophényl)sulfonamidoéthyl]indan-1-yl]hept-5-ènoïque, sel de sodium

Préparé selon le même mode opératoire que celui décrit dans l'exemple 15 en utilisant les produits de départ correspondants.
Rendement : 91 %
Point de fusion : 95-105°C
Résonance Magnétique Nucléaire du Proton (DMSO d6) :
Constante de couplage entre les protons éthyléniques cis : J = 10,8 Hz

| Microanalyse élémentaire : | | | | | |
|---|---|---|---|---|---|
| | C % | H % | N % | Cl % | S % |
| calculé | 59,56 | 5,62 | 2,89 | 7,33 | 6,62 |
| trouvé | 59,69 | 5,90 | 3,03 | 7,33 | 6,93 |

### Exemple 18 : Acide (4Z)-6-[2-[2-(4-chlorophényl)sulfonamidoéthyl]-1,2,3,4-tétrahydronaphtalen-2-yl]hex-4-ènoïque, sel de sodium

### Stade A : 1,2,3,4-Tétrahydronaphtalène-2-spiro-4'-(3',5'-dicyano-2',6'-dioxopipéridine), sel d'ammonium

Un mélange de 0,13 mole de 2-tétralone et de 0,26 mole de cyanoacétate d'éthyle dans 250 ml d'éthanol ammoniacal est maintenu à 0°C pendant 48 heures. Le solide est filtré, lavé à l'aide d'un minimum d'éthanol puis à l'éther et séché.
Rendement : 31 %

La suite de la synthèse est identique à celle utilisée pour l'exemple 15.
Rendement : 72°C
Point de fusion : 160-161°C
Résonance Magnétique Nucléaire du Proton (DMSO d6) :
Constante de couplage entre les protons éthyléniaues cis : J = 10,8 Hz

| Microanalyse élémentaire : | | | | | |
|---|---|---|---|---|---|
| | C % | H % | N % | Cl % | S % |
| calculé | 59,56 | 5,62 | 2,89 | 7,33 | 6,62 |
| trouvé | 59,57 | 5,48 | 3,07 | 7,28 | 6,64 |

### Exemple 19 : Acide (4Z)-6-[2-[2-(4-chlorophényl)sulfonamidoéthyl]indan-2-yl]hex-4-ènoïque, sel de sodium

### Stade A : Ester éthylique de l'acide cyanoindan-2-ylidène acétique

Préparé à partir d'indanone-2 et de cyanoacétate d'éthyle selon un mode opératoire identique à celui utilisé au stade A de l'exemple 15.
Rendement : 68 %
Point de fusion : 116°C

### Stade B : Acide 2-carboxy-2-indanacétique

A une suspension de 0,25 mole du composé préparé au stade précédent dans 310 ml d'éthanol, ajouter une solution de 0,2 mole de cyanure de potassium dans 310 ml d'eau. Dès que le milieu est devenu homogène, porter au reflux ½ heure. Concentrer le milieu réactionnel, ajouter au résidu 220 ml d'acide acétique glacial et 450 ml d'acide chlorhydrique concentré. Porter au reflux 48 heures. Concentrer le milieu réactionnel, diluer le résidu obtenu dans 150 ml d'eau et extraire le milieu à l'aide de 2 fois 100 ml d'acétate d'éthyle. Laver les phases organiques réunies à la soude 5N. Acidifier à pH = 1 la phase aqueuse par de l'acide chlorhydrique concentré, filtrer le précipité.
Rendement : 67 %
Point de fusion : 165°C

Les stades C à J sont identiques aux stades D à K de l'exemple 15.
Rendement : 70 %
Point de fusion : 100-105°C (décomposition)
Résonance Magnétique Nucléaire du Proton (DMSO d6) :
Constante de couplage entre les Drotons éthyléniques cis : J = 11,1 Hz

### Exemple 20 : Acide (5Z)-7-[[2-(4-chlorophényl)sulfonamidoéthyl]indan-2-yl]hept-5-ènoïque, sel de sodium

Préparé selon le procédé décrit dans l'exemple 23.
Rendement : 76 %
Point de fusion : 85-89°C
Résonance Magnétique Nucléaire du Proton (DMSO d6) :
Constante de couplage entre les protons éthvléniaues cis : J = 11,1 Hz

### Exemple 21 : Acide 6-[4-[2-(4-chlorophénylsulfonylamido)éthyl]-1-(4- chlorophénylsulfonyl)pipéridin-4-yl]hex-4-ènoïque, sel de sodium

Préparé selon le procédé décrit dans l'exemple 1.

### Etude pharmacologique des dérivés de l'invention

### Exemple 22 : Agrégation plaquettaire chez le lapin

Les lapins (2-3 kg) sont anesthésiés avec du sodium pentobarbital (30 mg/kg i.v.). Après cannulation de l'artère carotide gauche, le sang est prélevé sur citrate de sodium (0.109 M) (1 vol. de citrate pour 9 vol. de sang).

Le plasma riche en plaquettes (PRP) est obtenu par centrifugation (20°C) à 250 g pendant 20 minutes et le plasma pauvre en plaquettes (PPP) par centrifugation à 1000 g (10 min). Le nombre des plaquettes (PL) dans le PRP est ajusté entre 300-350000 PL/mm³ par dilution avec du PPP autologue. Le PRP est conservé à la température de la pièce jusqu'au moment du test et est utilisé dans les 4 heures qui suivent le prélèvement.

L'agrégation plaquettaire est réalisée à 37°C dans des tubes en verre siliconés à l'aide d'un agrégomètre. Le PRP et les PL sont agités à 1000 rpm (révolution par minute). Afin d'étudier l'activité des antagonistes du thromboxane, le PRP est incubé 1 min à 37°C, puis l'antagoniste est ajouté pour une durée de 3 min avant l'addition de l'agoniste U46619 (1.2 µM). Le volume final de la cuve est alors de 250 µl. L'intensité de l'agrégation plaquettaire est établie en prenant l'amplitude maximale de tracés agrégants et est exprimée en pourcentage de transmission lumineuse (% T). L'activité des antagonistes est exprimée en IC₅₀, c'est-à-dire la concentration de la substance qui induit 50 % d'inhibition de la réponse agrégante induite par le U46619.

Lors de ce test, les IC₅₀ des composés des exemples 2 et 6 sont égales à 2.10-⁷M.

### Exemple 23 : Agrégation plaquettaire chez le chien :

Après anesthésie de l'animal par le sodium pentobarbital (30 mg/kg i.v.), le sang artériel est prélevé sur citrate de sodium (0,109 M) (1 vol. de citrate pour 9 vol. de sang). Le plasma riche en plaquettes (PRP) est obtenu après centrifugation (20°C) à 200 g pendant 10 minutes. Le nombre de plaquettes dans le PRP est en moyenne de 300 000 PL/mm³. Le PRP est conservé à la température de la pièce jusqu'au moment du test et est utilisé dans les 4 heures qui suivent le prélèvement.

Les plaquettes du chien répondent faiblement au U46619 seul. L'addition d'adrénaline, qui seule n'induit pas d'agrégation, permet d'obtenir une réponse agrégante plus importante au U46619. Le PRP est incubé à 37°C en présence de l'antagoniste à tester pendant 3 minutes. L'agrégation est ensuite obtenue par l'addition de l'adrénaline (10 µM) suivie de celle du U46619 (1,2 µM) 30 secondes après. L'effet des antagonistes est mesuré et l'IC₅₀ est déterminée comme la concentration de l'antagoniste nécessaire pour produire 50 % d'inhibition des réponses agrégantes au U46619 + adrénaline.

### Lors de ce test, les IC₅₀ des composés sont les suivantes :

- Exemple 1: 1,4.10⁻⁷ M
- Exemple 2: 1,2.10⁻⁷ M
- Exemple 3: 6.10⁻⁸ M
- Exemple 4: 2.10⁻⁷ M
- Exemple 5: 1,4.10⁻⁷ M
- Exemple 12: 1,5.10⁻⁷ M

### Exemple 24 : Agrégation plaquettaire chez l'homme :

Le sang veineux est obtenu de volontaires humains n'ayant pas pris de l'aspirine pendant au moins 14 jours précédent l'expérience. Le sang est prélevé sur citrate de sodium (0,109 M) (1 vol. de citrate sur 9 vol. de sang). Le plasma riche en plaquettes (PRP) est obtenu après centrifugation (20°C) à 200 g pendant 10 minutes. Le nombre de plaquettes est en moyenne de 250000 PL/mm³. Le PRP est conservé à la température de la pièce jusqu'au moment du test et est utilisé dans les 2 heures qui suivent le prélèvement. Les antagonistes sont testés suivant la procédure décrite dans l'exemple 23.

Lors de ce test, les IC₅₀ des composés sont les suivantes :
- Exemple 2: 2.10⁻⁷ M
- Exemple 3: 8.10⁻⁸ M
- Exemple 6: 8.10⁻⁸ M
- Exemple 7: 3.10⁻⁷ M
- Exemple 8: 3.10⁻⁷ M
- Exemple 9: 2.10⁻⁷ M
- Exemple 12: 5.10⁻⁷ M
- Exemple 14: 8.10⁻⁷ M

### Exemple 25 : Détermination des valeurs des pA₂ sur la trachée de cobaye

Des cobayes mâles albinos de 400-500 grammes ont été sacrifiés par un coup sur la nuque et par élongation cervicale. La gorge est ouverte et la trachée est rapidement prélevée et puis découpée en anneaux de deux cartilages. Ces anneaux sont montés entre deux crochets dans des cuves thermostatées à 37°C contenant du liquide physiologique (composition en mM:NaCl 118 ; NaHCO₃ 25 ; Glucose 10 ; KCl 4.7 ; CaCl₂ 1.25 ; MgSO₄ 1.19 ; KH₂PO₄ 1.14).

La solution physiologique est bullée par un mélange de 95 % 02 - 5 % CO₂. Le crochet inférieur constitue le point fixe tandis que le crochet supérieur est relié à un capteur de force isométrique. Les tissus sont mis sous une tension de base de 3,5 grammes. Les substances pharmacologiques étudiées sont préparées immédiatement avant l'usage. Les drogues sont solubilisées dans l'eau ou dans le diméthylsulfoxyde.

Après le montage, les préparations sont laissées en repos pendant 90 minutes, des rinçages étant effectués toutes les 30 minutes. Après réajustement de la tension de base, une contraction provoquée par une dose unique d'agoniste (U46619 ; 10⁻⁵M) est provoquée afin de régulariser les contractions suivantes. Après lavage et retour à la ligne de base, une première courbe effet/concentration est réalisée par adjonction de doses cumulatives d'U46619 (10⁻⁹M à 10⁻⁵M ; l'espacement entre les doses est d'un demi-log). Cette première expérience permet de calculer la concentration efficace 50 % (EC₅₀) "témoin".

Cette EC₅₀ est calculée en routine de la manière suivante : les valeurs de tension sont d'abord converties en pourcentages par rapport à l'effet maximum, ces pourcentages sont ensuite reportés sur un graphique avec les pourcentages en ordonnée et les log (concentration) en abscisse. Une régression linéaire est alors effectuée sur les points compris entre 10 % et 90 % (ce qui correspond à la partie linéaire de la courbe sigmoïde). La concentration correspondant au demi effet maximum (50 %) peut être facilement calculée à l'aide des paramètres de la droite.

Après lavage et retour à la ligne de base, l'organe est mis en contact avec l'antagoniste (8 concentrations différentes pour chaque organe) pendant 20 minutes. Une deuxième courbe effet/concentration est alors réalisée en présence de l'antagoniste et l'EC₅₀ "traité" peut alors être calculée. On dispose ainsi de tous les éléments permettant le calcul du pA₂ (antagonisme compétitif) ou pD2 (antagonisme non compétitif).

Le pA₂ (qui représente le logarithme négatif de la concentration d'antagoniste en présence de laquelle il faut 2 fois plus d'agoniste pour obtenir le même effet) est déterminé en reportant sur un graphe les valeurs de log ((L/l)-1) par rapport à log (concentration d'antagoniste) avec L = effet en présence d'antagoniste et l = effet témoin.

Lors de ce test, les valeurs des pA₂ des composés de l'invention sont les suivantes :

| | |
|---|---|
| Exemple 1 : 9,4 | |
| Exemple 2 : 8,4 | |
| Exemple 3 : 9,3 | |
| Exemple 4 : 8,5 | |
| Exemple 5 : 8,2 | |
| Exemple 6 : 9,14 | Exemple 10 : 7,65 |
| Exemple 7 : 9,4 | Exemple 11 : 7,70 |
| Exemple 8 : 9,63 | Exemple 12 : 8,98 |
| Exemple 9 : 9,08 | Exemple 16 : 8,40 |

### Exemple 26: IC₅₀ sur la pression trachéale chez le cobaye

Des cobayes albinos mâles (350-400 g) soumis à une diète hydrique de 18 heures sont anesthésiés au carbamate d'éthyle (1.25 g/kg i.p.). Un cathéter est introduit dans l'artère carotide afin de mesurer la pression artérielle au moyen d'une cellule de pression. Un deuxième cathéter est introduit dans la veine jugulaire et sert à injecter les substances pharmacologiques. La trachée est cannulée et le cobaye est mis en respiration assistée au moyen d'un respirateur. La température de l'animal est maintenue à 37°C à l'aide d'une couverture thermostatée. Une aiguille piquée dans la cannule trachéale est reliée à une cellule de pression et permet d'enregistrer la pression trachéale.

Les cobayes sont prétraités par la d-tubocurarine (1 mg/kg i.v.) et par l'indométhacine (10 mg/kg i.v.). Injectée à la dose de 2 µg/kg i.v., le U46619 provoque une bronchoconstriction qui entraine une augmentation de la pression trachéale et induit une augmentation de la pression artérielle. Les réponses au U46619 sont réversibles et reproductibles si les injections sont effectuées toutes les 10 minutes.

Les antagonistes des récepteurs au thromboxane sont injectés 5 minutes avant les injections d'U46619. La dose d'antagoniste inhibant de 50 % l'augmentation de la pression trachéale causée par le U46619 est recherchée (IC₅₀).

Lors de ce test, les valeurs des IC₅₀ des composés de l'invention sont les suivantes :

| | |
|---|---|
| Exemple 1 : 27 µg/kg | Exemple 6 : 21 µg/kg |
| Exemple 2 : 120 µg/kg | Exemple 7 : 20 µg/kg |
| Exemple 3 : 19 µg/kg | Exemple 8 : 7 µg/kg |
| Exemple 4 : 105 µg/kg | Exemple 9 : 11 µg/kg |
| Exemple 5 : 66 µg/kg | Exemple 12 : 9 µg/kg |

### Exemple 27 : Composition pharmaceutique

Formule de préparation pour 1000 comprimés dosés à 10 mg

| | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 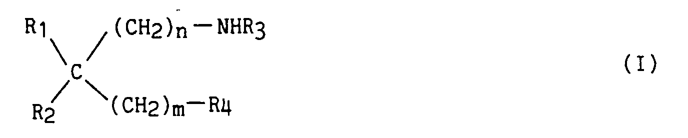 dans laquelle :
R₁ ou R₂ identiques ou différents représentent un radical alkyle (C₁-C₆) linéaire ou ramifié, un radical phényle (substitué ou non par un ou plusieurs atomes d'halogène ou radical alkyle (C₁-C₆) linéaire ou ramifié, alkoxy (C₁-C₆) linéaire ou ramifié, hydroxy, trihalogénométhyle), un radical pyridinyl, un radical thiényl,
ou forment, avec l'atome de carbone auxquels ils sont attachés, un cycle cycloalkyle (C₄-C₇) (substitué ou non par un radical alkyle (C₁-C₆) linéaire ou ramifié), un cycle benzocycloalkyle (C₄-C₇), ou un cycle pipéridin-4-yl (substitué ou non sur l'azote de la pipéridine par groupement phénylsulfonyl lui-même éventuellement substitué par un ou plusieurs atomes d'halogène ou groupements alkyle),
R₃ représente
- un radical phénylsulfonyl substitué ou non sur le noyau phényle par un atome d'halogène ou radical alkyle (C₁-C₆) linéaire ou ramifié,
- un radical naphtylsulfonyl,
- un radical alkyle (C₁-C₆) linéaire ou ramifié,
- un radical alkylaminocarbonyl,
- ou un radical acyle (C₁-C₆) linéaire ou ramifié,
R₄ représente l'un quelconque des radicaux :
―CH=CH―(CH₂)ₚ―CO₂H ou ―CH₂―CH₂―(CH₂)ₚ―CO₂H
dans lesquels p est égal à 0, 1, 2 ou 3,
n et m, identiques ou différents, représentent 0, 1 ou 2,
leurs isomères, énantiomères, diastéréoisomères et épimères ainsi que leurs sels d'addition à un acide ou à une base pharmaceutiquement acceptable.

2. Composés de formule (I) selon la revendication 1, dans laquelle R3 représente un groupement phénylsulfonyl substitué ou non sur le noyau phényle.

3. Composés de formule (I) selon la revendication 1, dans laquelle R₄ représente un radical -CH=CH-(CH₂)ₚ-CO₂H dans lequel p est égal à 0, 1, 2 ou 3.

4. Composés de formule (I) selon la revendication 3, dans laquelle la double liaison du radical R₄ est de configuration cis.

5. Composés de formule (I) selon la revendication 1 dans laquelle R₁ et R₂ forment avec l'atome de carbone auxquels ils sont attachés un cycle cycloalkyle (C₄-C₇) substitué ou non.

6. Composé de formule (I) selon la revendication 1 qui est l'acide (4Z)-8-[(4-chlorophényl)sulfonamido]-7,7-tétraméthylène oct-4-ènoïque.

7. Composé de formule (I) selon la revendication 1 qui est l'acide (5Z)-9-[(4-chlorophényl)sulfonamido]-8,8-tétraméthylène non-5-ènoïque.

8. Composé de formule (I) selon la revendication 1 qui est l'acide (4Z)-9-[(4-chlorophényl)sulfonamido]-8,8-tétraméthylène non-4-ènoïque.

9. Composé de formule (I) selon la revendication 1 qui est l'acide (5Z)-10-[(4-chlorophényl)sulfonamido]-8,8-tétraméthylène dèc-5-ènoïque.

10. Procédé de préparation des composés de formule (I) caractérisé en ce que l'on utilise comme produit de départ :
1/ un nitrile de formule (II) : 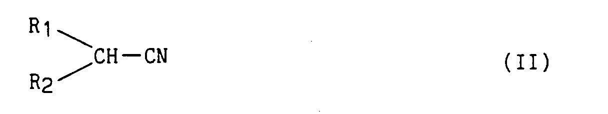 dans laquelle R₁ et R₂ ont la même signification que dans la formule (I), que l'on fait réagir :
sur le dérivé bromé de formule (III) en présence de diisopropylamidure de lithium dans du tetrahydrofurane : 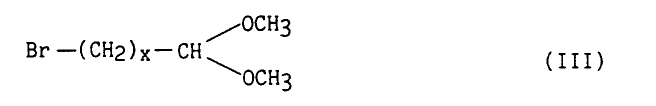 dans laquelle x représente n, n-1 ou m selon le dérivé de formule (I) que l'on souhaite obtenir,
pour conduire au dérivé de formule (IV) : 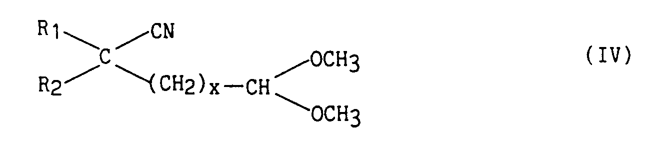 dans laquelle x a la même signification que précédemment,
soit que l'on réduit à l'aide de quatre équivalents d'hydrure de lithium aluminium dans de l'éther pour conduire au composé de formule (V) : 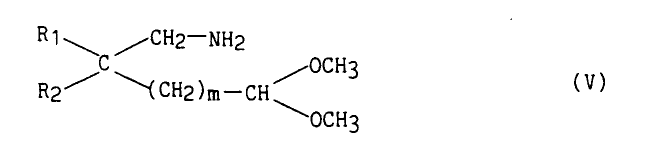 dans laquelle m a la même signification que dans la formule (I),
que l'on transforme en composé de formule (VII) : 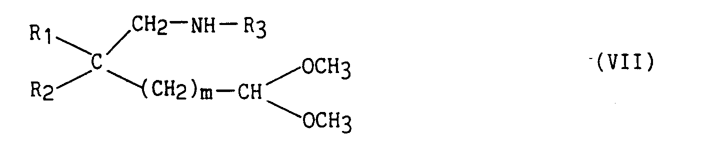 dans laquelle R₁, R₂ et m ont la même signification que dans la formule (I),
par réaction, selon la nature de R₃ :
* avec un dérivé halogéné de formule (VI) en présence de triéthylamine dans du toluène :
R'₃X (VI)
dans laquelle X représente un atome d'halogène,
R'₃ représente un radical phénylsulfonyl substitué ou non, ou un radical acyle,
* avec un isocyanate de formule R''-N=C=O (VI') dans laquelle R'' représente un radical alkyle et dans ce cas le radical R₃ de la formule (VII) est un radical alkylaminocarbonyl,
* un aldéhyde de formule R''₃ CHO (VI'') dans laquelle R''₃ est un radical alkyle pour former une imine qui est ensuite réduite en amine correspondante,
composé de formule (VII) :
ou bien que l'on transforme en aldéhyde de formule (VIIIa) en présence d'acide formique : 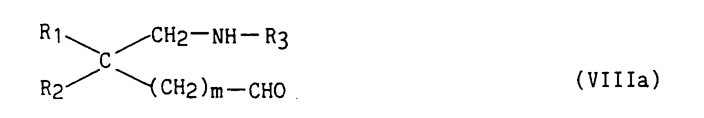 dans laquelle R₁, R₂ et m ont la même signification que dans la formule (I),
ou bien, lorsque m représente 1 ou 2, que l'on cyclise au reflux du toluène, en composé de formule (VIIIb), en présence d'acide paratoluène sulfonique : 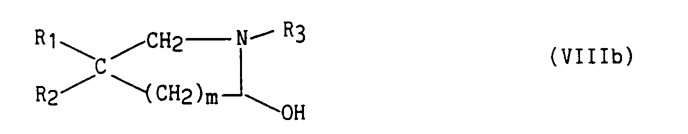 dans laquelle R₁, R₂, R₃ et m ont la même signification que précédemment,
composé de formule (VIIIa) ou (VIIIb), que l'on fait réagir avec l'ylure de phosphore de formule (IX), préparé par réaction du sel de phosphonium correspondant en présence de terbutanolate de potassium dans du tétrahydrofurane,
(C₆H₅)₃P=CH―(CH₂)ₚ―CO₂H (IX)
dans laquelle p a la même signification que dans la formule (I),
pour conduire au composé de formule (I/a), cas particulier des composés de formule (I) : 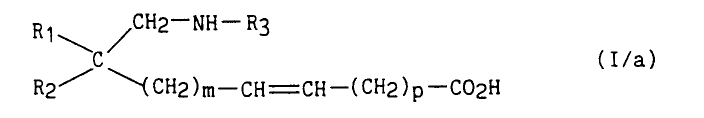
soit que l'on transforme en aldéhyde correspondant de formule (X) à l'aide de trois équivalents d'hydrure de diisobutylaluminium dans du toluène, 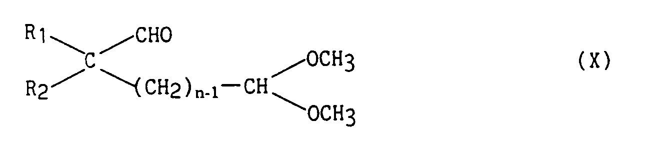 dans laquelle n a la même signification que dans la formule (I), que l'on fait réagir avec l'ylure de phosphore de formule (IX) défini précédemment,
pour conduire après traitement au diazomèthane au composé de formule (XI) : 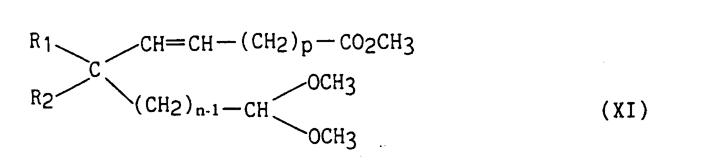 dans laquelle R₁, R₂, p et n ont la même signification que précédemment,
qui, après hydrolyse de l'acétal, réduction de l'aldéhyde formé en alcool, et formation de l'amine correspondante, est transformé en composé de formule (I/b), cas particulier des composés de formule (I), en faisant réagir soit le dérivé halogéné de formule (VI), soit l'isocyanate de formule (VI'), soit l'aldéhyde de formule (VI'') selon la nature du radical R₃ que l'on souhaite obtenir, et en saponifiant l'ester obtenu, 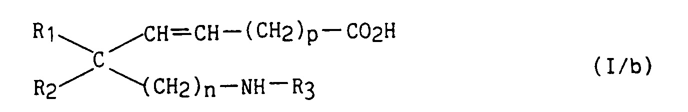
2/ un ester de formule XII) : 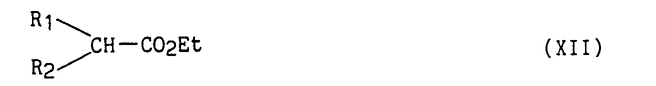 dans laquelle R₁ et R₂ ont la même signification que dans la formule (I), sur lequel on fait réagir le dérivé bromé de formule (III) défini précédemment,
pour conduire au composé de formule (XIII) : 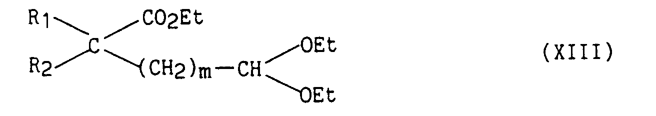 dans laquelle R₁ et R₂ ont la même signification que dans la formule (I), que l'on transforme :
a en amine correspondante de formule (XIV), via l'azoture, puis l'isocyanate correspondants ou via l'acide et la benzyloxy-carbonylamine correspondants, 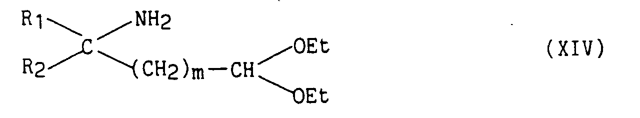 dans laquelle R₁ et R₂ ont la même signification que dans la formule (I),
sur laquelle on réalise les mêmes réactions que celles décrites précédemment, pour le passage du composé (V) au composé (VII),
pour conduire au composé de formule (XV) : 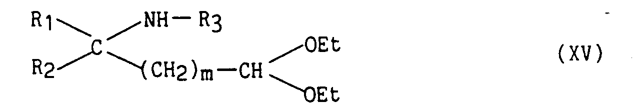 dans laquelle R₁, R₂ et m ont la même signification que dans la formule (I),
ou bien, que l'on transforme en aldéhyde de formule (XVIa) en présence d'acide formique : 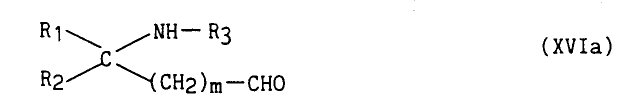 dans laquelle R₁ et R₂ ont la même signification que dans la formule (I),
ou bien, lorsque m = 2, que l'on cyclise au reflux du toluène en composé de formule (XVIb), en présence d'acide p.toluène sulfonique : 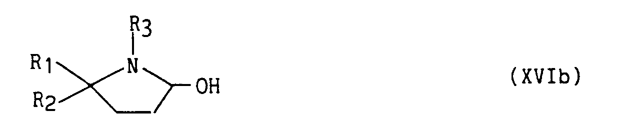 dans laquelle R₁, R₂ et R₃ ont la même signification que précédemment,
composé de formule (XVIa) ou (XVIb) que l'on fait réagir avec l'ylure de formule (IX) décrit précédemment,
pour conduire au composé de formule (I/c), cas particulier des composés de formule (I), 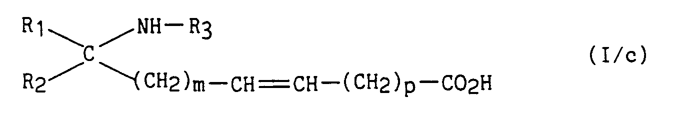 dans laquelle R₁, R₂, R₃ et m ont la même signification que dans la formule (I),
b en alcool correspondant de formule (XVII) à l'aide d'un excès d'hydrure de lithium aluminium, 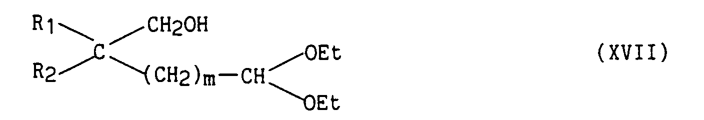 dans laquelle R₁, R₂ et m ont la même signification que dans la formule (I),
puis, après passage par le mésylate, est transformé en nitrile correspondant de formule (XVIII) : 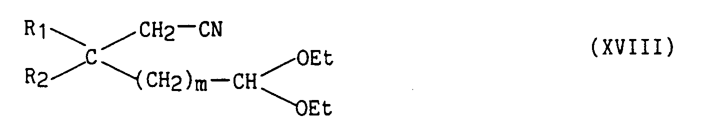 dans laquelle R₁, R₂ et m ont la même signification que dans la formule (I),
qui est réduit en amine correspondant de formule (XIX) : 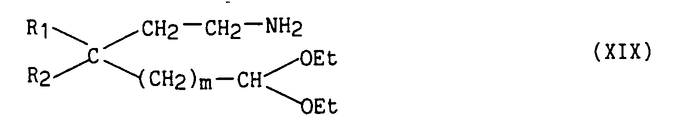 dans laquelle R₁, R₂ et m ont la même signification que dans la formule (I),
sur laquelle on réalise les mêmes réactions que celles décrites précédemment, pour le passage du composé (V) au composé (VII), pour conduire au composé de formule (XX) : 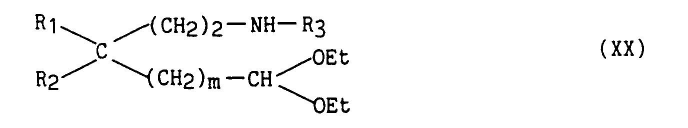 dans laquelle R₁, R₂, R₃ et m ont la même signification que dans la formule (I),
qui, soit après déprotection à l'acide formique, soit cyclisation au reflux du toluène en présence d'acide p.toluène sulfonique (lorsque m représente 1 ou 2), puis réaction de l'ylure de formule (IX) décrit précédemment, est transformé en composé de formule (I/d), cas particulier des composés de formule (I), 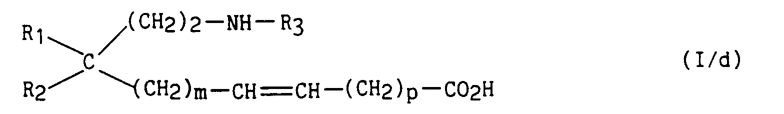 dans laquelle R₃, m et p ont la même signification que dans la formule (I),
- composés de formules (I/a), (I/b), (I/c) ou (I/d) que l'on réduit, si on le souhaite, par hydrogénation catalytique pour conduire respectivement aux composés de formule (I/a₁), (I/b₁), (I/c₁) ou (I/d₁), cas particulier des composés de formule (I), 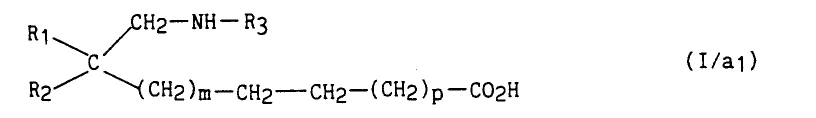 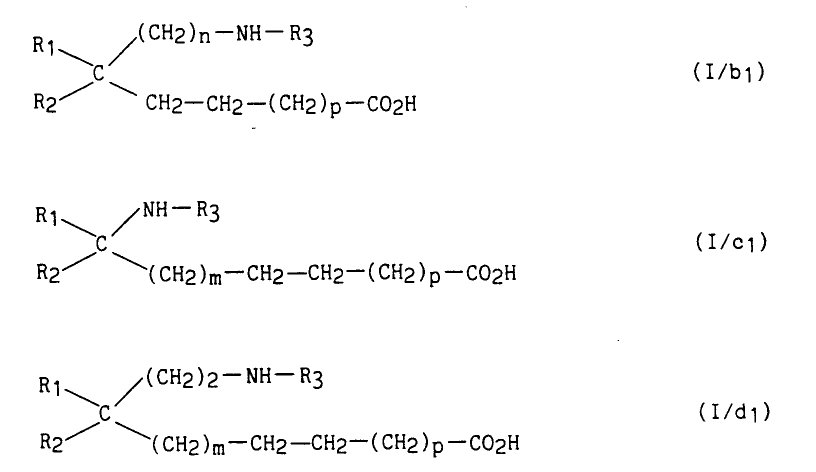 dans lesquelles R₁, R₂, m, n, p ont la même signification que dans la formule (I),
- composés de formules (I/a), (I/a₁), (I/b), (I/b₁), (I/c), (I/c₁), (I/d) ou (I/d₁) :
- que l'on purifie selon des techniques classiques de purification,
- dont on sépare, éventuellement, les isomères, selon des techniques classiques de séparation,
- et que l'on transforme, si on le souhaite, en leurs sels d'addition à un acide ou à une base pharmaceutiquement acceptable.

11. Procédé de préparation des composés répondant plus spécifiquement à la formule (I'), cas particulier des composés de formule (I) 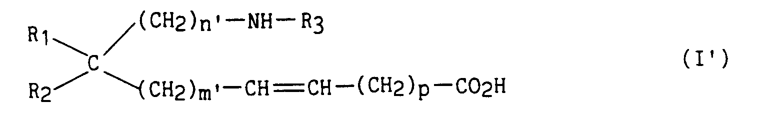 dans laquelle R₁, R₂, R₃ et p ont la même signification que dans la formule (I),
n' = 1, 2
m' = 1, 2
peuvent également être obtenus en utilisant le procédé caractérisé en ce que l'on utilise comme produit de départ un composé de formule (XXI) : 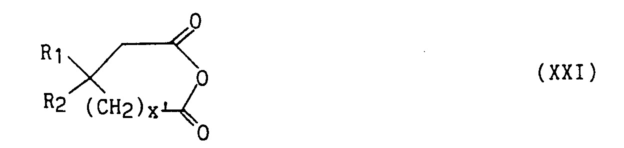 dans laquelle R₁ et R₂ ont la même signification que dans la formule (I') et x' est égal à 0 ou 1,
que l'on réduit en présence de borohydrure de sodium dans du méthanol pour conduire au composé de formule (XXII) : 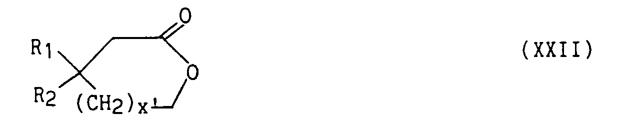 dans laquelle R₁, R₂ et x' ont la même signification que précédemment qui subit l'action :
soit de l'hydrure de diisobutylaluminium dans du toluène, pour conduire au composé de formule (XXIII) : 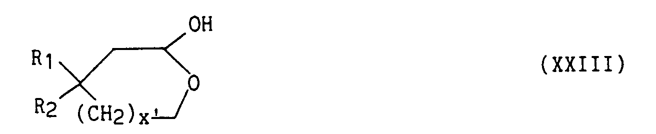 dans laquelle R₁, R₂ et x' ont la même signification que précédemment,
que l'on fait réagir avec l'ylure de phosphore de formule (IX) :
(C₆H₅)₃P=CH―(CH₂)ₚ―CO₂H (IX)
dans laquelle p a la même signification que dans la formule (I'),
pour conduire, après action du diazométhane, au composé de formule (XXIV) : 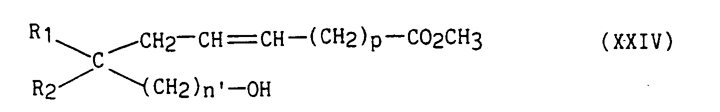 dans laquelle R₁, R₂, p et n' ont la même signification que dans la formule (I'),
que l'on fait réagir avec le chlorure de mésyle en présence de triéthylamine dans du toluène,
pour conduire au composé de formule (XXV) : 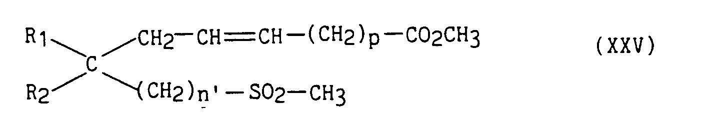 dans laquelle R₁, R₂, p et n' ont la même signification que dans la formule (I'),
que l'on fait réagir avec une solution de ditert-butyliminodicarbonate dans du diméthylformamide que l'on a fait préalablement réagir avec une suspension d'hydrure de sodium dans du diméthylformamide,
pour conduire au composé de formule (XXVI) : 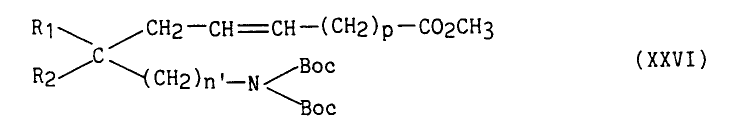 dans laquelle R₁, R₂, p et n' ont la même signification que dans (I'),
qui est transformé en amine correspondante de formule (XXVII) par réaction dans de l'éther chlorhydrique : 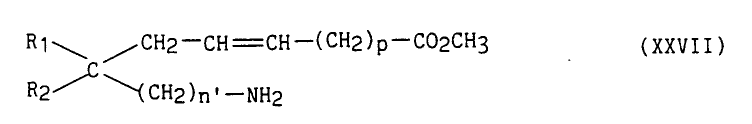 dans laquelle R₁, R₂, p et n' ont la même signification que dans la formule (I'),
que l'on transforme en composé de formule (XXVIII), 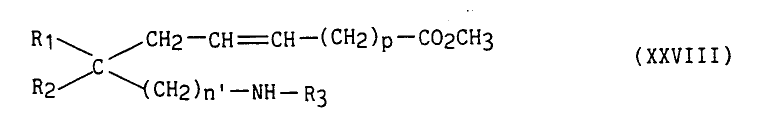 dans laquelle R₁, R₂, R₃, p et n' ont la même signification que dans la formule (I'),
par réaction, selon la nature de R₃
- soit avec un dérivé halogéné de formule (VI) en présence de triéthylamine dans du toluène :
R'₃X (VI)
dans laquelle X représente un atome d'halogène,
R'₃ représente un radical phénylsulfonyl substitué ou non, ou un radical acyle,
- soit avec un isocyanate de formule R''-N=C=O (VI') dans laquelle R'' représente un radical alkyle et dans ce cas le radical R₃ de la formule (VII) est un radical alkylaminocarbonyl,
- soit avec un aldéhyde de formule R''₃ CHO (VI'') dans laquelle R''₃ est un radical alkyle pour former une imine qui est ensuite réduite en amine correspondante,
que l'on saponifie pour conduire au composé de formule (I/e), cas particulier des composés de formule (I'), 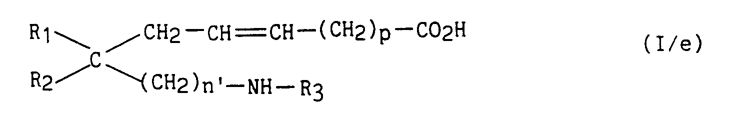 dans laquelle R₁, R₂, R₃, p et n' ont la même signification que dans la formule (I')
soit du cyanure de potassium dans du diméthylsulfoxyde pour conduire au composé de formule (XXIX) : 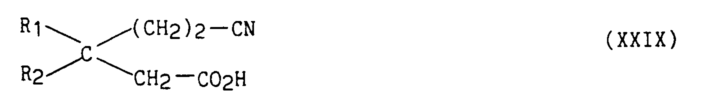 dans laquelle R₁ et R₂ ont la même signification que dans la formule (I'),
que l'on fait réagir avec du chloroformiate d'éthyle dans de l'acétone en présence de triéthylamine, pour conduire au composé de formule (XXX) : 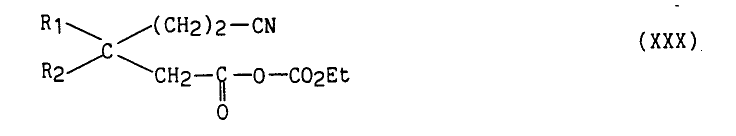 dans laquelle R₁ et R₂ ont la même signification que dans la formule (I'),
qui subit l'action de l'azoture de sodium en milieu aqueux, puis après chauffage dans du toluène l'action du tertbutanol,
pour conduire au composé de formule (XXXI) : 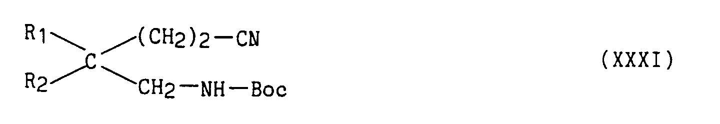 dans laquelle R₁ et R₂ ont la même signification que dans la formule (I'),
que l'on réduit en aldéhyde correspondant de formule (XXXII) en présence de trois équivalents d'hydrure de diisobutylaluminium dans du toluène : 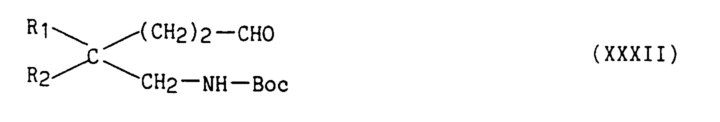 dans laquelle R₁ et R₂ ont la même signification que dans la formule (I'),
que l'on met en réaction avec l'ylure de phosphore de formule (IX) décrit précédemment,
pour conduire au composé de formule (XXXIII) : 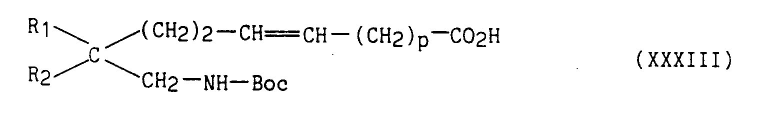 dans laquelle R₁, R₂ et p ont la même signification que dans la formule (I'),
dont la fonction amine est déprotégée dans de l'éther chlorhydrique, puis qui subit les mêmes réactions que celles décrites pour le passage du composé (XXVII) au composé (XXVIII),
pour conduire au composé de formule (I/f), cas particulier des composés de formule (I), 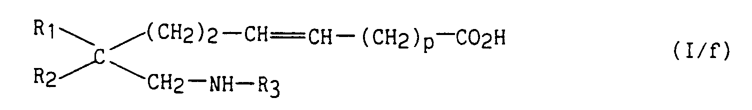 dans laquelle R₁, R₂, R₃ et p ont la même signification que dans la formule (I'),
- composés de formule (I/e) ou (I/f) que l'on réduit, si on le souhaite, par hydrogénation catalytique pour conduire respectivement aux composés de formule (I/e₁) ou (I/f₁), cas particulier des composés de formule (I) : 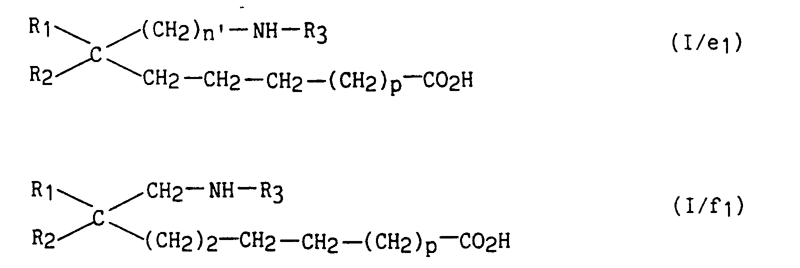 dans lesquelles R₁, R₂, p et n' ont la même signification que dans la formule (I),
- composés de formules (I/e), (I/e₁), (I/f) ou (I/f₁) :
- que l'on purifie selon des techniques classiques de purification,
- dont on sépare, éventuellement, les isomères, selon des techniques classiques de séparation,
- et que l'on transforme, si on le souhaite, en leurs sels d'addition à un acide ou à une base pharmaceutiquement acceptable.

12. Compositions pharmaceutiques contenant comme principe actif au moins un composé selon l'une quelconque des revendications 1 à 9 seul ou en combinaison avec un ou plusieurs véhicules inertes non toxiques, pharmaceutiquement acceptables.

13. Composition pharmaceutique selon la revendication 8 contenant au moins un principe actif selon l'une des revendications 1 à 9 utiles en tant qu'antagoniste des récepteurs au thromboxane A₂ ou en tant qu'inhibiteur de la thromboxane -A₂-synthase.

## Claims

1. Compounds of formula (I): 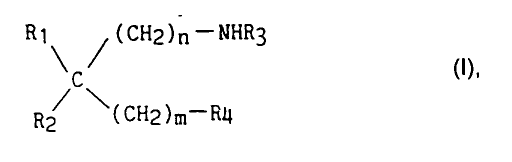 in which:
R₁ and R₂, which are the same or different, each represents a linear or branched (C₁-C₆)-alkyl radical, a phenyl radical (unsubstituted or substituted by one or more halogen atoms or radicals linear or branched (C₁-C₆)-alkyl, linear or branched (C₁-C₆)-alkoxy, hydroxy, trihalomethyl), a pyridinyl radical or a thienyl radical, or, together with the carbon atom to which they are attached, form a (C₄-C₇)-cycloalkyl ring (unsubstituted or substituted by a linear or branched (C₁-C₆)-alkyl radical), a benzo-(C₄-C₇)-cycloalkyl ring or a piperidin-4-yl ring (unsubstituted or substituted on the nitrogen of the piperidine by a phenylsulphonyl group that is itself optionally substituted by one or more halogen atoms or alkyl groups)
R₃ represents
- a phenylsulphonyl radical that is unsubstituted or substituted on the phenyl nucleus by a halogen atom or by a linear or branched (C₁-C₆)-alkyl radical,
- a naphthylsulphonyl radical,
- a linear or branched (C₁-C₆)-alkyl radical,
- an alkylaminocarbonyl radical,
- or a linear or branched (C₁-C₆)-acyl radical,
R₄ represents either one of the radicals:
―CH =CH―(CH₂)ₚ―CO₂H or ―CH₂―CH₂―(CH₂)ₚ―CO₂H
in which p is 0, 1, 2 or 3,
and
n and m, which are the same or different, each represents 0, 1 or 2,
their isomers, enantiomers, diastereoisomers and epimers, and addition salts thereof with a pharmaceutically acceptable acid or base.

2. Compounds of formula (I) according to claim 1, in which R₃ represents a phenylsulphonyl group that is unsubstituted or substituted on the phenyl nucleus.

3. Compounds of formula (I) according to claim 1, in which R₄ represents a -CH=CH-(CH₂)ₚ-CO₂H radical in which p is 0, 1, 2 or 3.

4. Compounds of formula (I) according to claim 3, in which the double bond of the radical R₄ is in the *cis* configuration.

5. Compounds of formula (I) according to claim 1, in which R₁ and R₂, together with the carbon atom to which they are attached, form an unsubstituted or substituted (C₄-C₇)-cycloalkyl ring.

6. Compound of formula (1) according to claim 1 that is (*4Z*)-8-[(4-chlorophenyl)-sulphonamido]-7,7-tetramethyleneoct-4-enoic acid.

7. Compound of formula (I) according to claim 1 that is (*5Z*)-9-[(4-chlorophenyl)-sulphonamido]-8,8-tetramethylenenon-5-enoic acid.

8. Compound of formula (1) according to claim 1 that is (*4Z*)-9-[(4-chlorophenyl)-sulphonamido]-8,8-tetramethylenenon-4-enoic acid.

9. Compound of formula (I) according to claim 1 that is (*5Z*)-10-[(4-chlorophenyl)-sulphonamido]-8,8-tetramethylenedec-5-enoic acid.

10. Process for the preparation of compounds of formula (I), characterised in that there is used as starting material:
1. a nitrile of formula (II): 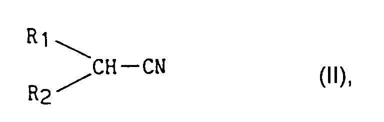 in which R₁ and R₂ are as defined for formula (I),
which is reacted:
with a brominated compound of formula (III) in the presence of lithium diisopropylamide in tetrahydrofuran: 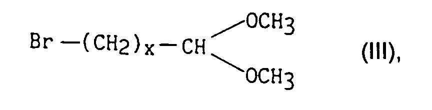 in which x represents n, n-1 or m according to the compound of formula (I) desired,
to yield a compound of formula (IV): 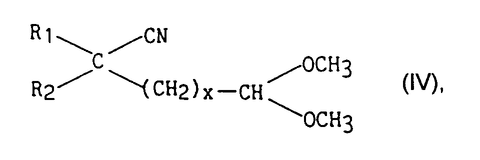 in which x is as defined above,
either which is reduced using four equivalents of lithium aluminium hydride in ether to yield a compound of formula (V): 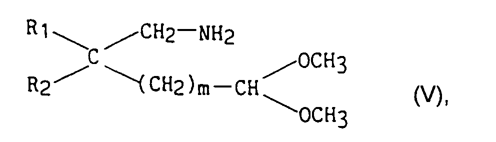 in which m is as defined for formula (I),
which is converted into a compound of formula (VII): 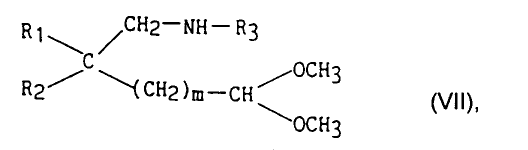 in which R₁, R₂ and m are as defined for formula (I),
by reaction, according to the nature of R₃:
* with a halogenated compound of formula (VI) in the presence of triethylamine in toluene:
R'₃X (VI),
in which X represents a halogen atom and
R'₃ represents an unsubstituted or substituted phenylsulphonyl radical or an acyl radical,
* with an isocyanate of formula R''-N=C=O (VI'), in which R'' represents an alkyl radical, and in that case the radical R₃ of formula (VII) is an alkylamino-carbonyl radical,
* with an aldehyde of formula R''₃CHO (VI''), in which R''₃ is an alkyl radical, to form an imine, which is then reduced to a corresponding amine,
which compound of formula (VII):
either is converted into an aldehyde of formula (VIIIa) in the presence of formic acid: 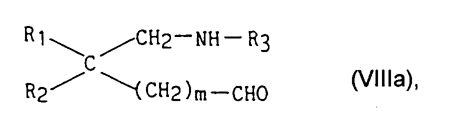 in which R₁, R₂ and m are as defined for formula (I),
or, when m represents 1 or 2, is cyclised by refluxing with toluene in the presence of *para*-toluenesulphonic acid to form a compound of formula (VIIIb): 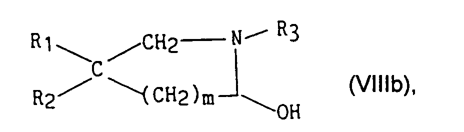 in which R₁, R₂, R₃ and m are as defined above,
which compound of formula (VIIIa) or (VIIIb) is reacted with a phosphorus ylid of formula (IX) prepared by reaction of the corresponding phosphonium salt in the presence of potassium *tert*-butanolate in tetrahydrofuran,
(C₆H₅)₃P=CH―(CH₂)ₚ―CO₂H (IX),
in which p is as defined for formula (I),
to yield a compound of formula (I/a), a particular case of the compounds of formula (I): 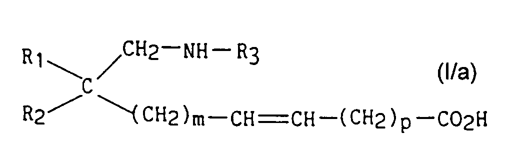
or which is converted into a corresponding aldehyde of formula (X) using three equivalents of diisobutylaluminium hydride in toluene 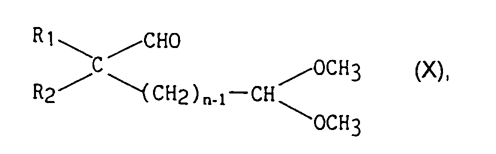 in which n is as defined for formula (I),
which is reacted with the above-defined phosphorus ylid of formula (IX)
to yield, after treatment with diazomethane, a compound of formula (XI): 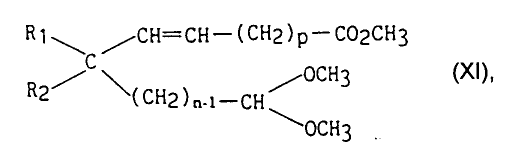 in which R_{1,} R₂, p and n are as defined above,
which, after hydrolysis of the acetal, reduction of the resulting aldehyde in alcohol and formation of the corresponding amine, is converted into a compound of formula (I/b), a particular case of the compounds of formula (I), by reacting a halogenated compound of formula (VI) or an isocyanate of formula (VI') or an aldehyde of formula (VI'') according to the nature of the radical R₃ desired, and hydrolysing the ester obtained 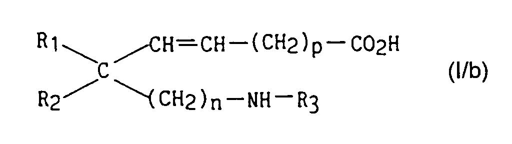
2. an ester of formula (XII): 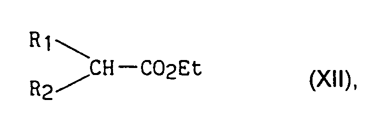 in which R₁ and R₂ are as defined for formula (I),
which is reacted with the brominated compound of formula (III) defined above
to yield a compound of formula (XIII): 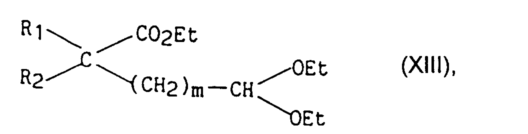 in which R₁ and R₂ are as defined for formula (I),
which is converted:
a into a corresponding amine of formula (XIV), *via* the corresponding azide and then the corresponding isocyanate or *via* the corresponding acid and the corresponding benzyloxycarbonylamine, 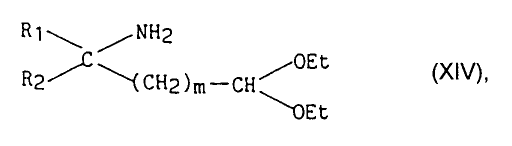 in which R₁ and R₂ are as defined for formula (I),
on which the same reactions are performed as those described previously for the route from compound (V) to compound (VII),
to yield a compound of formula (XV): 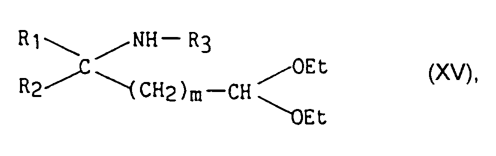 in which R₁, R₂ and m are as defined for formula (I),
either which is converted into an aldehyde of formula (XVIa) in the presence of formic acid: 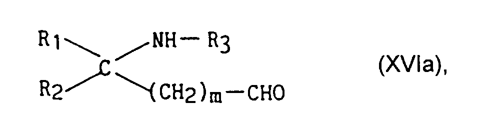 in which R₁ and R₂ are as defined for formula (I),
or which, when m = 2, is cyclised, by refluxing in toluene in the presence of *p*-toluene-sulphonic acid, to form a compound of formula (XVIb): 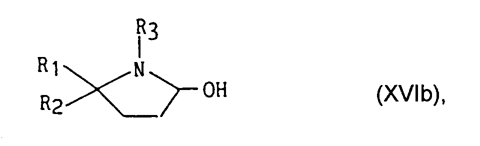 in which R₁, R₂ and R₃ are as defined above,
which compound of formula (XVIa) or (XVIb) is reacted with the ylid of formula (IX) described previously,
to yield a compound of formula (I/c), a particular case of the compounds of formula (I), 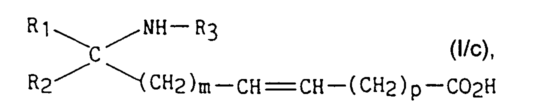 in which R₁, R₂, R₃ and m are as defined for formula (I),
b into a corresponding alcohol of formula (XVII) with the aid of an excess of lithium aluminium hydride, 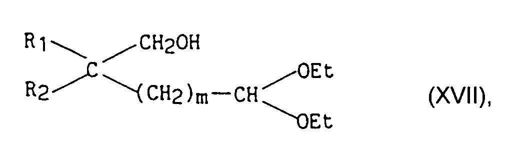 in which R₁, R₂ and m are as defined for formula (I),
then, *via* the mesylate, is converted into a corresponding nitrile of formula (XVIII): 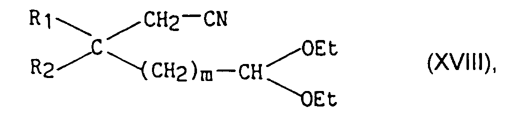 in which R₁, R₂ and m are as defined for formula (I),
which is reduced to form a corresponding amine of formula (XIX): 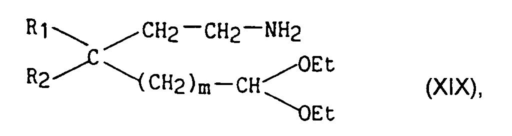 in which R₁, R₂ and m are as defined for formula (I),
on which the same reactions are performed as those described previously for the route from compound (V) to compound (VII),
to yield a compound of formula (XX): 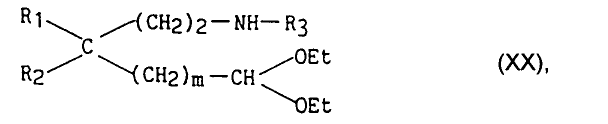 in which R₁, R₂, R₃ and m are as defined for formula (I),
which, after either deprotection using formic acid or cyclisation by refluxing with toluene in the presence of *p*-toluenesulphonic acid (when m represents 1 or 2) and then reaction of the ylid of formula (IX) described previously, is converted into a compound of formula (I/d), a particular case of the compounds of formula (I), 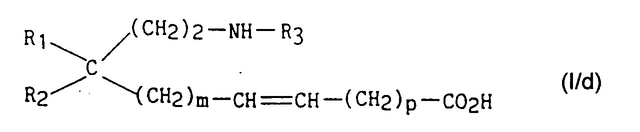 in which R₃, m and p are as defined for formula (I),
- which compounds of formulae (I/a), (I/b), (I/c) and (I/d) are reduced, if desired, by catalytic hydrogenation to yield compounds of formulae (I/a₁), (I/b₁), (I/c₁) and (I/d₁), respectively, particular cases of the compounds of formula (I), 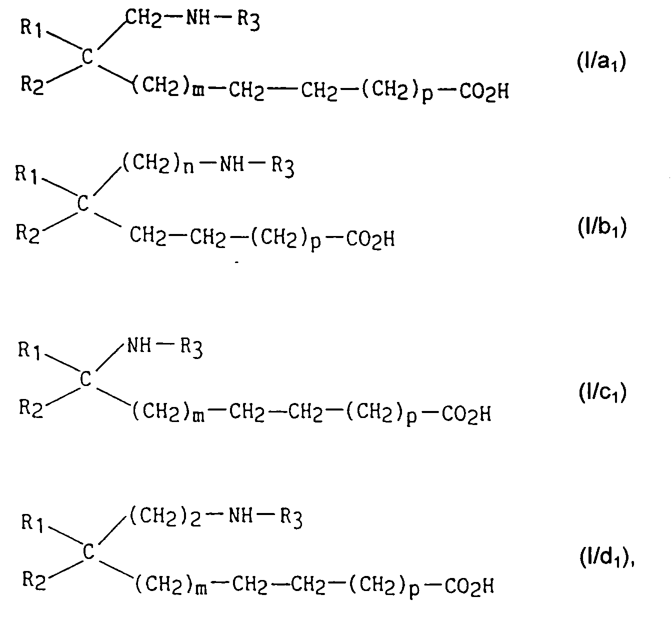 in which R₁, R₂, m, n and p are as defined for formula (I),
- which compounds of formulae (I/a), (I/a₁), (I/b), (I/b₁), (I/c), (I/c₁), (I/d) and (I/d₁):
- are purified according to customary purification techniques,
- are separated, if appropriate, into their isomers according to customary separation techniques,
- and are converted, if desired, into their addition salts with a pharmaceutically acceptable acid or base.

11. Process for the preparation of compounds corresponding more especially to formula (I'), a particular case of the compounds of formula (I) 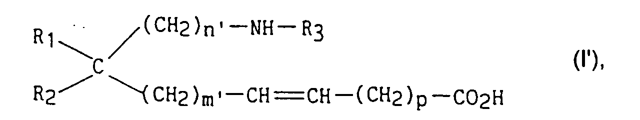 in which R₁, R₂, R₃ and p are as defined for formula (I),
n' = 1 or 2
m' = 1 or 2,
using the process characterised in that there is used as starting material a compound of formula (XXI): 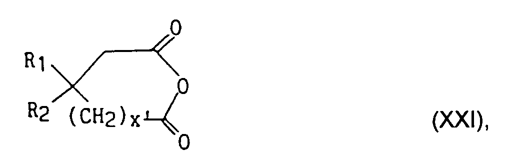 in which R₁ and R₂ are as defined for formula (I') and x' is 0 or 1,
which is reduced in the presence of sodium borohydride in methanol to yield a compound of formula (XXII): 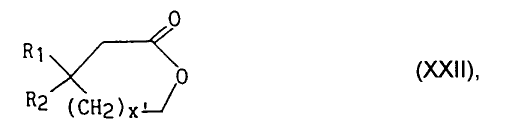 in which R₁, R₂ and x' are as defined above, which is subjected to the action:
either of diisobutylaluminium hydride in toluene to yield a compound of formula (XXIII): 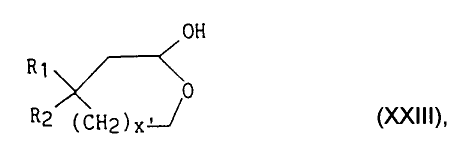 in which R₁, R₂ and x' are as defined above,
which is reacted with a phosphorus ylid of formula (IX):
(C₆H₅)₃P=CH―(CH₂)ₚ―CO₂H (IX),
in which p is as defined for formula (I'),
to yield, after the action of diazomethane, a compound of formula (XXIV): 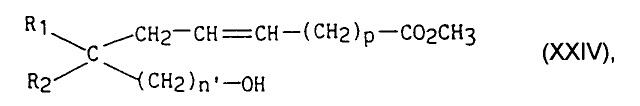 in which R₁, R₂, p and n' are as defined for formula (I'),
which is reacted with mesyl chloride in the presence of triethylamine in toluene
to yield a compound of formula (XXV): 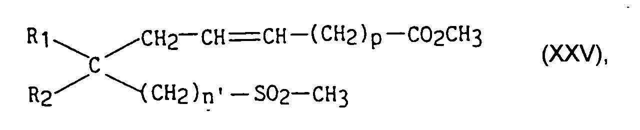 in which R₁, R₂, p and n' are as defined for formula (I'),
which is reacted with a solution of di-*tert*-butyliminodicarbonate in dimethylformamide which has previously been reacted with a suspension of sodium hydride in dimethylformamide,
to yield a compound of formula (XXVI): 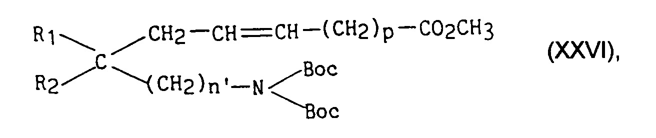 in which R₁, R₂, p and n' are as defined for (I'),
which is converted into a corresponding amine of formula (XXVII) by reaction in ethereal hydrogen chloride: 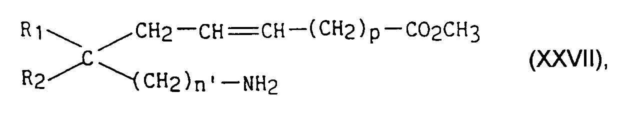 in which R₁, R₂, p and n' are as defined for formula (I'),
which is converted into a compound of formula (XXVIII) 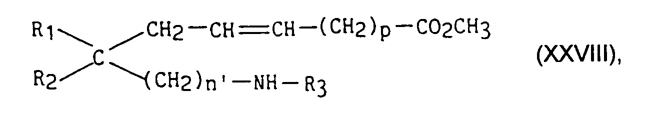 in which R₁, R₂, R₃, p and n' are as defined for formula (I'),
by reaction, according to the nature of R₃,
- either with a halogenated compound of formula (VI) in the presence of triethylamine in toluene:
R'₃X (VI),
in which X represents a halogen atom and
R'₃ represents an unsubstituted or substituted phenylsulphonyl radical or an acyl radical,
- or with an isocyanate of formula R''-N=C=O (VI'), in which R'' represents an alkyl radical and in that case the radical R₃ of formula (VII) is an alkylaminocarbonyl radical,
- or with an aldehyde of formula R''₃CHO (VI''), in which R''₃ is an alkyl radical, to form an mine which is then reduced to a corresponding amine,
which is hydrolysed to yield a compound of formula (I/e), a particular case of the compounds of formula (I'), 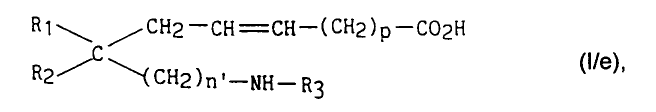 in which R₁, R₂, R₃, p and n' are as defined for formula (I')
or of potassium cyanide in dimethyl sulphoxide to yield a compound of formula (XXIX): 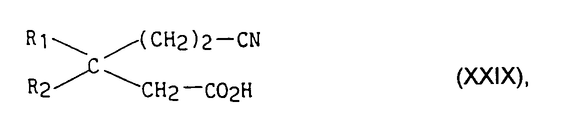 in which R₁ and R₂ are as defined for formula (I'),
which is reacted with ethyl chloroformate in acetone in the presence of triethylamine to yield a compound of formula (XXX): 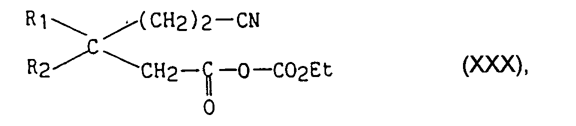 in which R₁ and R₂ are as defined for formula (I'),
which is subjected to the action of sodium azide in an aqueous medium and then, after heating in toluene, to the action of *tert*-butanol
to yield a compound of formula (XXXI): 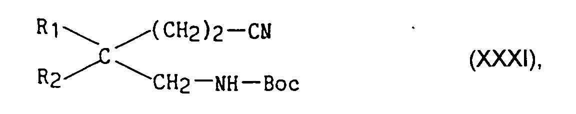 in which R₁ and R₂ are as defined for formula (I'),
which is reduced to a corresponding aldehyde of formula (XXXII) in the presence of three equivalents of diisobutylaluminium hydride in toluene: 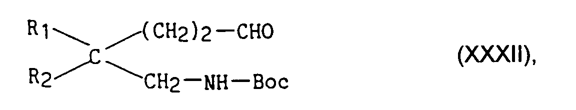 in which R₁ and R₂ are as defined for formula (I'),
which is reacted with the phosphorus ylid of formula (IX) described previously, to yield a compound of formula (XXXIII): 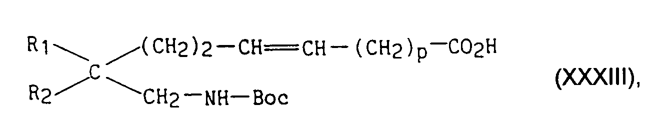 in which R₁, R₂ and p are as defined for formula (I'),
the amine function of which is deprotected in ethereal hydrogen chloride, and which is then subjected to the same reactions as those described for the route from compound (XXVII) to compound (XXVIII)
to yield a compound of formula (I/f), a particular case of the compounds of formula (I), 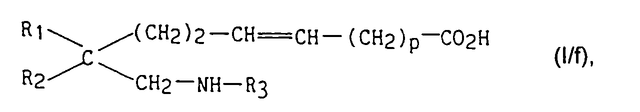 in which R₁, R₂, R₃ and p are as defined for formula (I'),
- which compounds of formulae (I/e) and (I/f) are reduced, if desired, by catalytic hydrogenation to yield compounds of formulae (I/e₁) and (I/f₁), respectively, particular cases of the compounds of formula (I), 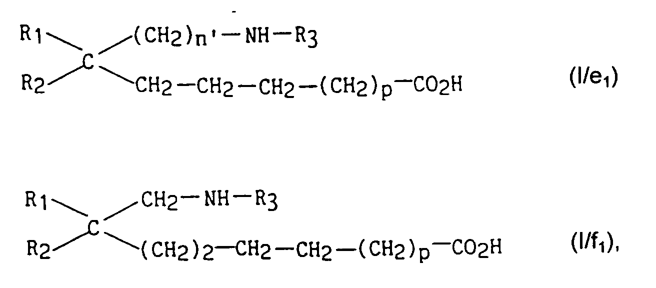 in which R₁, R₂, p and n' are as defined for formula (I),
- which compounds of formulae (I/e), (I/e₁), (I/f) and (I/f₁):
- are purified according to customary purification techniques,
- are separated, if appropriate, into their isomers according to customary separation techniques,
- and are converted, if desired, into their addition salts with a pharmaceutically acceptable acid or base.

12. Pharmaceutical compositions comprising as active ingredient at least one compound according to any one of claims 1 to 9 alone or in combination with one or more pharmaceutically acceptable, inert, non-toxic carriers.

13. Pharmaceutical composition according to claim 8 comprising at least one active ingredient according to any one of claims 1 to 9 for use as a thromboxane A₂ receptor antagonist or as a thromboxane A₂ synthase inhibitor.

## Patentansprüche

1. Verbindungen der Formel (I): 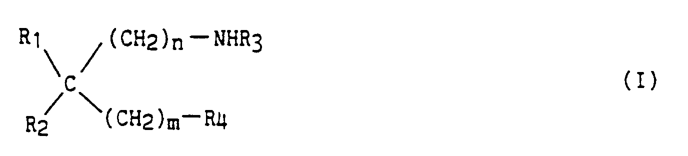 in der:
R₁ oder R₂, die gleichartig oder verschieden sind, eine geradkettige oder verzweigte (C₁-C₆)-Alkylgruppe, eine Phenylgruppe (die gegebenenfalls durch eines oder mehrere Halogenatome oder geradkettige oder verzweigte (C₁-C₆)-Alkylgruppen, geradkettige oder verzweigte (C₁-C₆)-Alkoxygruppen, Hydroxylgruppen oder Trihalogenmethylgruppen substituiert ist), eine Pyridinylgruppe oder eine Thienylgruppe
oder zusammen mit dem Kohlenstoffatom, an das sie gebunden sind, einen (C₄-C₇)-Cycloalkylring (welcher gegebenenfalls durch eine geradkettige oder verzweigte (C₁-C₆)-Alkylgruppe substituiert ist), einen Benzo-(C₄-C₇)-cycloalkylring oder einen Piperidin-4-yl-ring (der gegebenenfalls am Stickstoffatom des Piperidins durch eine Phenylsulfonylgruppe substituiert ist, die ihrerseits gegebenenfalls durch eines oder mehrere Halogenatome oder Alkylgruppen substituiert ist),
R₃
- eine Phenylsulfonylgruppe, die gegebenenfalls am Phenylkern durch ein Halogenatom oder eine geradkettige oder verzweigte (C₁-C₆)-Alkylgruppe substituiert ist,
- eine Naphthylsulfonylgruppe,
- eine geradkettige oder verzweigte (C₁-C₆)-Alkylgruppe,
- eine Alkylaminocarbonylgruppe oder
- eine geradkettige oder verzweigte (C₁-C₆)-Acylgruppe.
R₄ eine der folgenden Gruppen:
- CH = CH - (CH₂)ₚ - CO₂H oder - CH₂ - CH₂ - (CH₂)ₚ - CO₂H
worin p 0, 1, 2 oder 3 darstellt,
und n und m, die gleichartig oder verschieden sein können, 0, 1 oder 2 bedeuten, deren Isomere, Enantiomere, Diastereoisomere und Epimere sowie deren Additionssalze mit einer pharmazeutisch annehmbaren Säure oder Base.

2. Verbindungen der Formel (I) nach Anspruch 1, in der R₃ eine gegebenenfalls am Phenylkern substituierte Phenylsulfonylgruppe bedeutet.

3. Verbindungen der Formel (I) nach Anspruch 1, in der R₄ eine Gruppe -CH=CH-(CH₂)ₚ-CO₂H darstellt, in der p 0, 1, 2 oder 3 bedeutet.

4. Verbindungen der Formel (I) nach Anspruch 3, bei der die Doppelbindung der Gruppe R₃ in der cis-Konfiguration steht.

5. Verbindungen der Formel (I) nach Anspruch 1, in der R₁ und R₂ zusammen mit dem Kohlenstoffatom, an das sie gebunden sind, einen gegebenenfalls substituierten (C₄-C₇)-Cycloalkylring bilden.

6. Verbindung der Formel (I) nach Anspruch 1, nämlich (4Z)-8-[(4-Chlorphenyl)-sulfonamido]-7,7-tetramethylen-oct-4-ensäure.

7. Verbindung der Formel (I) nach Anspruch 1, nämlich (5Z)-9-[(4-Chlorphenyl)-sulfonamido]-8,8-tetramethylen-non-5-ensäure.

8. Verbindung der Formel (I) nach Anspruch 1, nämlich (4Z)-9-[(4-Chlorphenyl)-sulfonamidol-8,8-tetramethylen-non-4-ensäure.

9. Verbindung der Formel (I) nach Anspruch 1, nämlich (5Z)-10-[(4-Chlorphenyl)-sulfonamido]-8,8-tetramethylen-dec-5-ensäure.

10. Verfahren zur Herstellung der Verbindungen der Formel (I), dadurch gekennzeichnet, daß man als Ausgangsprodukt:
1/ ein Nitril der Formel (II) verwendet: 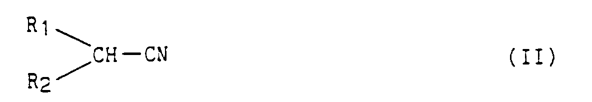 in der R₁ und R₂ die bezüglich der Formel (I) angegebenen Bedeutungen besitzen, welches man:
in Gegenwart von Lithiumdiisopropylamid in Tetrahydrofuran auf das Bromderivat der Formel (III) einwirken läßt: 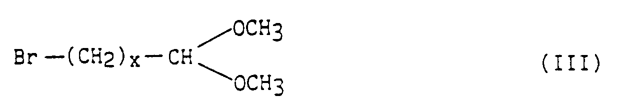 in der x in Abhängigkeit von dem herzustellenden Derivat der Formel (I) n, n-1 oder m bedeutet,
zur Bildung des Derivats der Formel (IV): 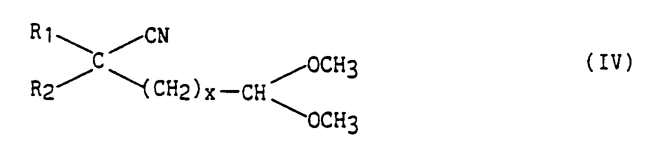 in der x die oben angegebene Bedeutung besitzt, welches man
entweder mit Hilfe von vier Äquivalenten Lithiumaluminiumhydrid in Ether reduziert zur Bildung der Verbindung der Formel (V): 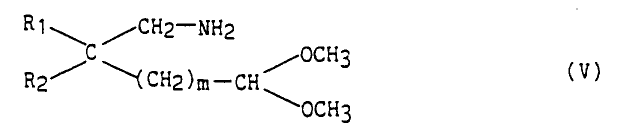 in der m die bezüglich der Formel (I) angegebenen Bedeutungen besitzt,
welche man in die Verbindung der Formel (VII): 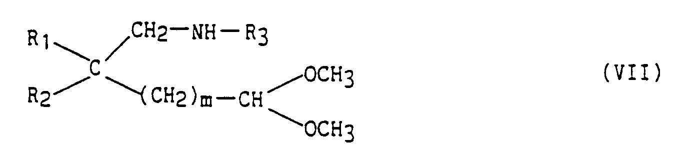 in der R₁, R₂ und m die bezüglich der Formel (I) angegebenen Bedeutungen besitzen, umwandelt
durch Reaktion in Abhängigkeit von der Art von R₃:
* mit einem Halogenderivat der Formel (VI) in Gegenwart von Triethylamin in Toluol:
R'₃X (VI)
in der X einHalogenatom darstellt und
R'₃ eine gegebenenfalls substituierte Phenylsulfonylgruppe oder eine Acylgruppe darstellt,
* mit einem Isocyanat der Formel R'' - N = C = O (VI'), in der R'' eine Alkylgruppe darstellt und in diesem Fall die Gruppe R₃ der Formel (VII) eine Alkylaminocarbonylgruppe ist,
* mit einem Aldehyd der Formel R''₃ CHO (VI''), in der R''₃ eine Alkylgruppe darstellt, zur Bildung eines Imins, welches anschließend zu dem entsprechenden Amin reduziert wird,
welche Verbindung der Formel (VII) man:
entweder in Gegenwart von Ameisensäure in den Aldehyd der Formel (VIIIa) umwandelt: 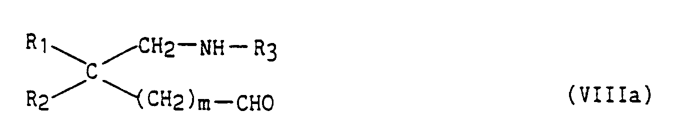 in der R₁, R₂ und m die bezüglich der Formel (I) angegebenen Bedeutungen besitzen,
oder, wenn m 1 oder 2 bedeutet, in Gegenwart von p-Toluolsulfonsäure in Toluol am Rückfluß zu der Verbindung der Formel (VIIIb) cyclisiert: 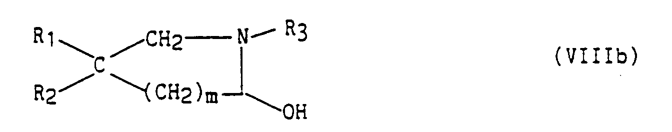 in der R₁, R₂, R₃ und m die oben angegebenen Bedeutungen besitzen,
welche Verbindung der Formel (VIIIa) oder (VIIIb) man mit dem durch Reaktion des entsprechenden Phosphoniumsalzes hergestellten Phosphorylid der Formel (IX):
(C₆H₅)₃P = CH - (CH₂)ₚ - CO₂H (IX)
in der p die bezuglich der Formel (I) angegebenen Bedeutungen besitzt,
in Gegenwart von Kalium-tert.-butanolat in Tetrahydrofuran umsetzt zur Bildung der Verbindung der Formel (I/a), einem Sonderfall der Verbindungen der Formel (I): 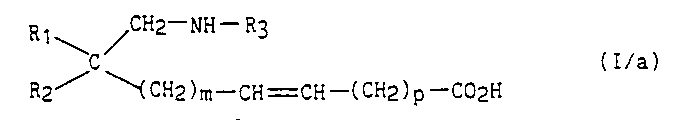
oder welches man mit Hilfe von drei Äquivalenten Diisobutylaluminiumhydrid in Toluol in den entsprechenden Aldehyd der Formel (X) umwandelt: 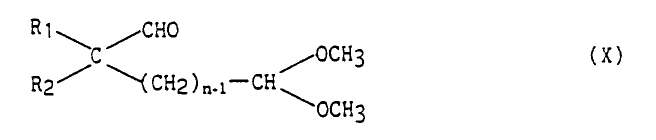 in der n die bezüglich der Formel (I) angegebenen Bedeutungen besitzt,
welchen man mit dem oben definierten Phosphorylid der Formel (IX) umsetzt,
so daß man nach der Behandlung mit Diazomethan die Verbindung der Formel (XI) erhält: 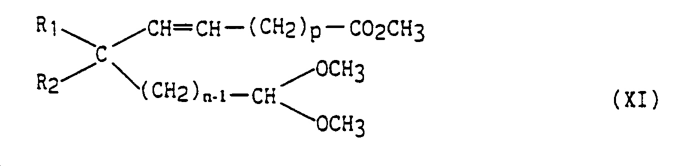 in der R₁, R₂, p und n die oben angegebenen Bedeutungen besitzen,
welche man nach der Hydrolyse zu dem Acetal, der Reduktion des gebildeten Aldehyds zu dem Alkohol und der Bildung des entsprechenden Amins in die Verbindung der Formel (I/b), einem Sonderfall der Verbindungen der Formel (I),umwandelt: 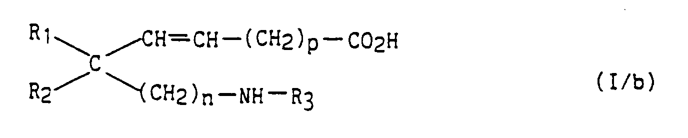 indem man entweder das Halogenderivat der Formel (VI) oder das Isocyanat der Formel (VI') oder den Aldehyd der Formel (VI'') in Abhängigkeit von der herzustellenden Gruppe R₃ zusetzt und den erhaltenen Ester verseift,
2/ einen Ester der Formel (XII) verwendet: 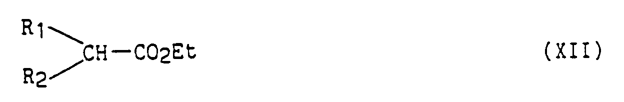 in der R₁ und R₂ die bezüglich der Formel (I) angegebenen Bedeutungen besitzen,
welchen man mit dem oben definierten Bromderivat der Formel (III) umsetzt zur Bildung der Verbindung der Formel (XIII): 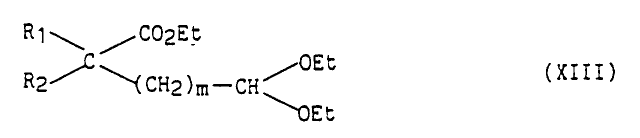 in der R₁ und R₂ die bezuglich der Formel (I) angegebenen Bedeutungen besitzen,
welche man:
a über das entsprechende Azid und dann das entsprechende Isocyanat oder über die Säure und das entsprechenden Benzyloxycarbonylamin in das entsprechende Amin der Formel (XIV): 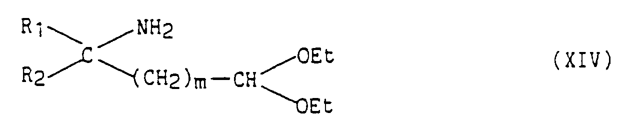 in der R₁ und R₂ die bezuglich der Formel (I) angegebenen Bedeutungen besitzen, umwandelt,
welches man den gleichen Reaktionen unterwirft, welche oben für die Umwandlung der Verbindung (V) in die Verbindung (VII) beschrieben sind,
so daß man zu der Verbindung der Formel (XV) gelangt: 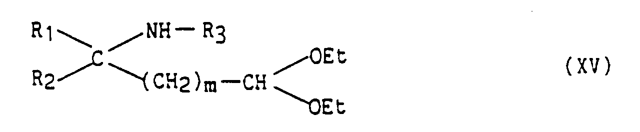 in der R₁, R₂ und m die oben angegebenen Bedeutungen besitzen,
welche man entweder in Gegenwart von Ameisensäure in den Aldehyd der Formel (XVIa) umwandelt: 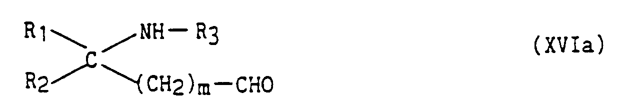 in der R₁ und R₂ die bezuglich der Formel (I) angegebenen Bedeutungen besitzen,
oder, wenn m = 2 bedeutet, man am Rückfluß in Toluol in Gegenwart von p-Toluolsulionsäure zu der Verbindung der Formel (XVIb) cyclisiert: 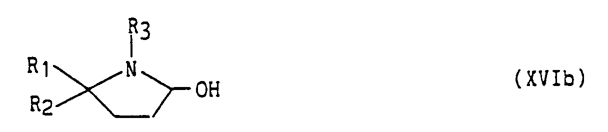 in der R₁, R₂ und R₃ die oben angegebenen Bedeutungen besitzen,
welche Verbindung der Formel (XVIa) oder (XVIb) man mit dem oben beschriebenen Ylid der Formel (IX) umsetzt,
so daß man die Verbindung der Formel (I/c) erhält, einem Sonderfall der Verbindungen der Formel (I): 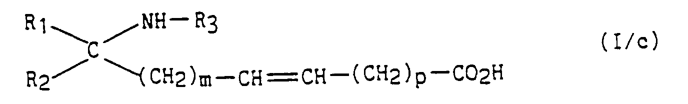 in der R₁, R₂, R₃ und m die bezuglich der Formel (I) angegebenen Bedeutungen besitzen,
b mit Hilfe eines Überschusses von Lithiumaluminiumhydrid in den entsprechenden Alkohol der Formel (XVII) umwandelt: 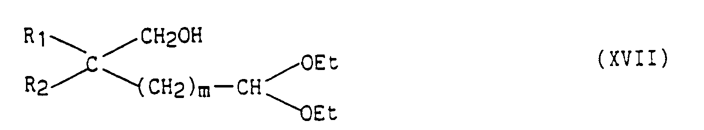 in der R₁, R₂ und m die bezuglich der Formel (I) angegebenen Bedeutungen besitzen,
welchen man nach der Überführung in das Mesylat in das entsprechende Nitril der Formel (XVIII) umwandelt: 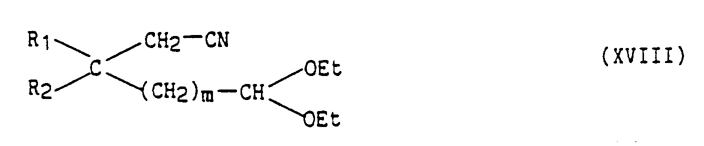 in der R₁, R₂ und m die bezüglich der Formel (I) angegebenen Bedeutungen besitzen,
welches man zu dem entsprechenden Amin der Formel (XIX) reduziert: 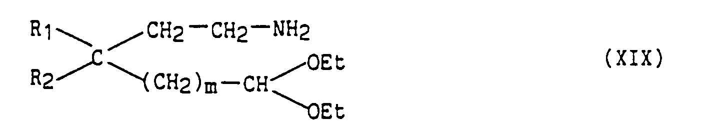 in der R₁, R₂ und m die bezüglich der Formel (I) angegebenen Bedeutungen besitzen,
welches man den gleichen Reaktionen unterwirft, wie sie oben für die Umwandlung der Verbindung (V) in die Verbindung (VII) beschrieben worden sind,
zur Bildung der Verbindung der Formel (XX): 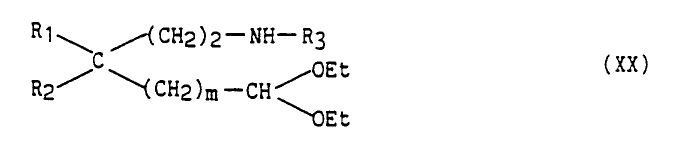 in der R₁, R₂, R₃ und m die bezüglich der Formel (I) angegebenen Bedeutungen besitzen,
welche nach der Abspaltung der Schutzgruppe mit Hilfe von Ameisensäure oder nach der Cyclisierung in Gegenwart von p-Toluolsulfonsäure in Toluol am Rückfluß (wenn m 1 oder 2 bedeutet) und dann durch Reaktion des oben beschriebenen Ylids der Formel (IX) in die Verbindung der Formel (I/d) umgewandelt wird, einem Sonderfall der Verbindungen der Formel (I): 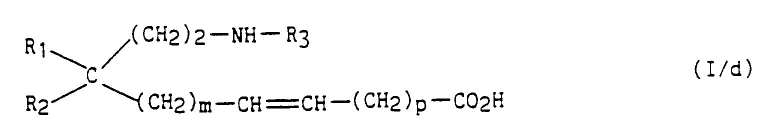 In der R₃, m und p die bezüglich der Formel (I) angegebenen Bedeutungen besitzen,
- welche Verbindungen der Formeln (I/a), (I/b), (I/c) oder (I/d) man gewünschtenfalls durch katalytische Hydrierung reduziert, so daß man zu den Verbindungen der Formeln (I/a₁), (I/b₁), (I/c₁) oder (I/d₁) gelangt, einem Sonderfall der Verbindungen der Formel (I): 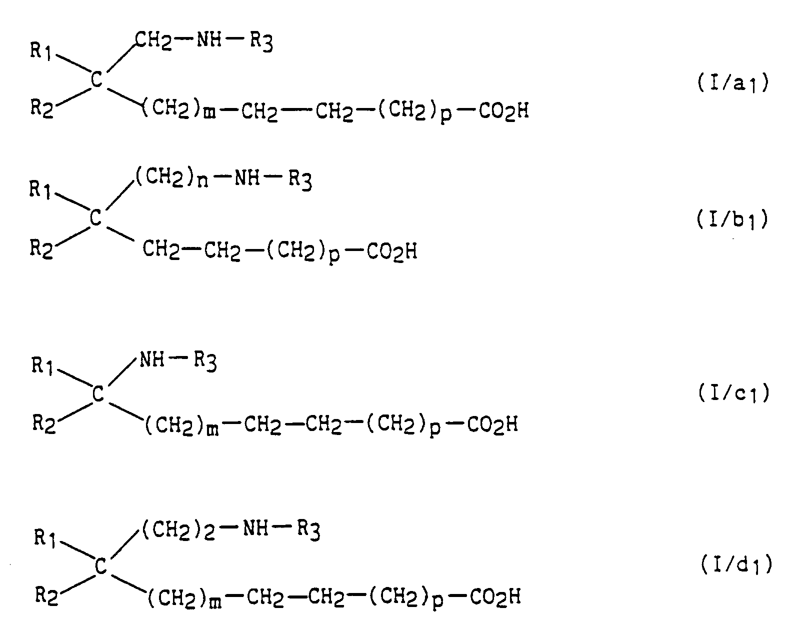 worin R₁, R₂, m, n und p die bezüglich der Formel (I) angegebenen Bedeutungen besitzen,
- welche Verbindungen der Formeln (I/a), (I/a₁), (I/b), (I/b₁), (I/c), (I/c₁), (I/d) oder (I/d₁):
- man mit Hilfe klassischer Reinigungsverfahren reinigt,
- gegebenenfalls unter Anwendung klassischer Trennverfahren in ihre Isomeren auftrennt und
- gewünschtenfalls mit einer pharmazeutisch annehmbaren Säure oder Base in ihre Additionssalze umwandelt.

11. Verfahren zur Herstellung der Verbindungen der Formel (I'), einem Sonderfall der Verbindungen der Formel (I): 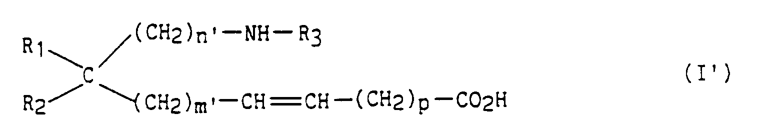 In der R₁, R₂, R₃ und p die bezüglich der Formel (I) angegebenen Bedeutungen besitzen,
n' = 1 oder 2
m' = 1 oder 2
bedeuten,
welche ebenfalls unter Anwendung eines Verfahrens erhalten werden können, das dadurch gekennzeichnet ist, daß man als Ausgangsprodukt eine Verbindung der Formel (XXI) verwendet: 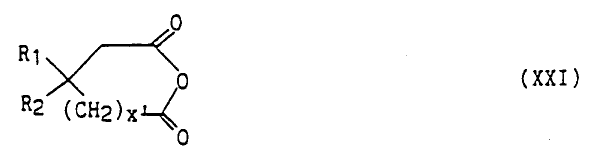 in der R₁ und R₂ die bezüglich der Formel (I') angegebenen Bedeutungen besitzen und x' 0 oder 1 darstellt,
welches man in Gegenwart von Natriumborhydrid in Methanol reduziert zur Bildung der Verbindung der Formel (XXII): 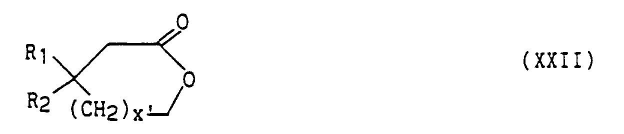 in der R₁, R₂ und x' die oben angegebenen Bedeutungen besitzen, welche man:
entweder der Einwirkung von Diisobutylaluminlumhydrid In Toluol unterwirft zur Bildung der Verbindung der Formel (XXIII): 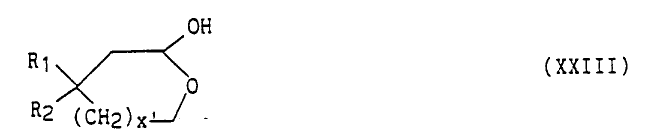 in der R₁, R₂ und x' die oben angegebenen Bedeutungen besitzen,
welche man mit dem Phosphorylid der Formel (IX):
(C₆H₅)₃P = CH - (CH₂)ₚ - CO₂H (IX)
in der p die bezüglich der Formel (I') angegebenen Bedeutungen besitzt,
umsetzt, so daß man nach der Einwirkung von Diazomethan die Verbindung der Formel (XXIV) erhält: 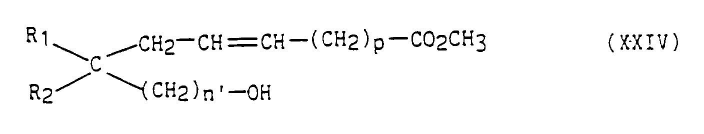 in der R₁, R₂, p und n' die bezüglich der Formel (I') angegebenen Bedeutungen besitzen,
welche man mit Mesylchlorid in Gegenwart von Triethylamin in Toluol umsetzt zur Bildung der Verbindung der Formel (XXV): 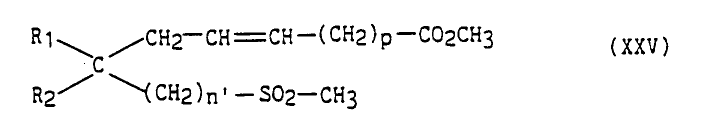 in der R₁, R₂, p und n' die bezüglich der Formel (I') angegebenen Bedeutungen besitzen,
welche man mit einer Lösung von Di-tert.-butyliminodicarbonat in Dimethylformamid, welche man zuvor mit einer Suspension von Natriumhydrid in Dimethylformamid umgesetzt hat, reagieren läßt,
so daß man zu der Verbindung der Formel (XXVI) gelangt: 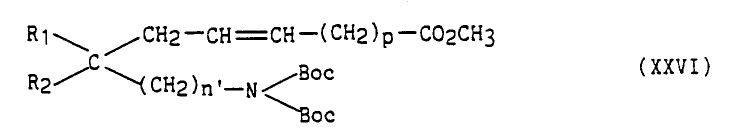 in der R₁, R₂, p und n' die bezüglich der Formel (I') angegebenen Bedeutungen besitzen,
welche durch Reaktion in Chlorwasserstoff-haltigem Ether in das entsprechende Amin der Formel (XXVII) umgewandelt wird: 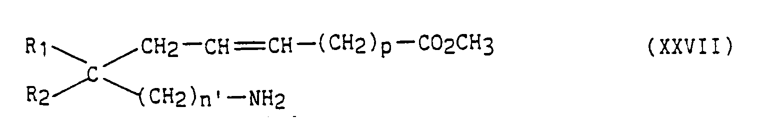 in der R₁, R₂, p und n' die bezüglich der Formel (I') angegebenen Bedeutungen besitzen,
welches man in die Verbindung der Formel (XXVIII): 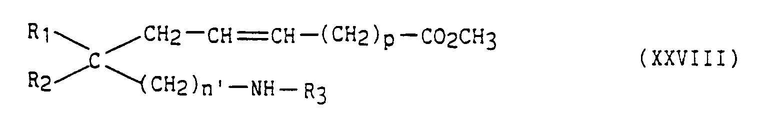 in der R₁, R₂, R₃, p und n' die bezüglich der Formel (I') angegebenen Bedeutungen besitzen, umwandelt
durch Reaktion in Abhängigkeit von der Art von R₃,
- entweder in Gegenwart von Triethylamin in Toluol mit einem Halogenderivat der Formel (VI) umsetzt:
R'₃X (VI)
in der X ein Halogenatom und R'₃ eine gegebenenfalls substituierte Phenylsulfonylgruppe oder eine Acylgruppe bedeuten,
- oder mit einem Isocyanat der Formel R''-N=C=O (VI') umsetzt, in der R'' eine Alkylgruppe und in diesem Fall die Gruppe R₃ der Formel (VII) eine Alkylaminocarbonylgruppe bedeuten,
- oder mit einem Aldehyd der Formel R''₃ CHO (VI'') umsetzt, in der R''₃ eine Alkylgruppe bedeutet, zur Bildung eines Imins, welches anschließend zu dem entsprechenden Amin reduziert wird,
welches man verseift, so daß man zu der Verbindung der Formel (I/e) gelangt, einem Sonderfall der Verbindungen der Formel (I'): 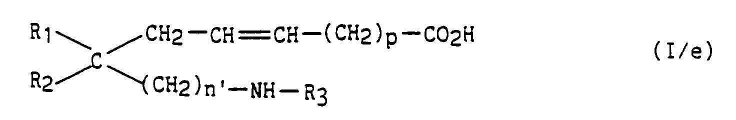 in der R₁, R₂, R₃, p und n' die bezüglich der Formel (I') angegebenen Bedeutungen besitzen,
oder der Einwirkung von Kaliumcyanid in Dimethylsulfoxid unterwirft zur Bildung der Verbindung der Formel (XXIX): 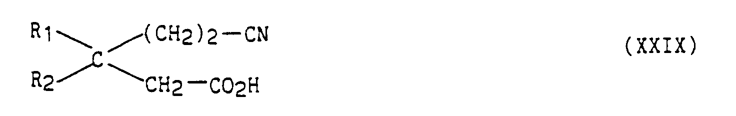 in der R₁ und R₂ die bezüglich der Formel (I') angegebenen Bedeutungen besitzen,
welche man mit Chlorameisensäureethylester in Aceton in Gegenwart von Triethylamin umsetzt zur Bildung der Verbindung der Formel (XXX): 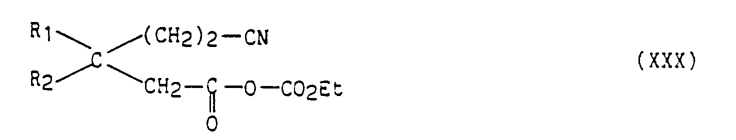 in der R₁ und R₂ die bezüglich der Formel (I') angegebenen Bedeutungen besitzen,
welche man der Einwirkung von Natriumazid in wäßrigem Medium und dann nach dem Erhitzen in Toluol der Einwirkung von tert.-Butanol unterzieht
zur Bildung der Verbindung der Formel (XXXI): 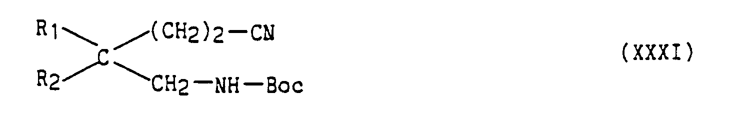 in der R₁ und R₂ die bezüglich der Formel (I') angegebenen Bedeutungen besitzen,
welche man in Gegenwart von drei Äquivalenten Diisobutylaluminiumhydrid in Toluol zu dem entsprechenden Aldehyd der Formel (XXXII) reduziert: 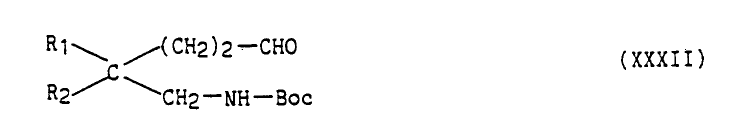 in der R₁ und R₂ die bezüglich der Formel (I') angegebenen Bedeutungen besitzen,
welchen man mit dem oben beschriebenen Phosphorylid der Formel (IX) umsetzt
zur Bildung der Verbindung der Formel (XXXIII): 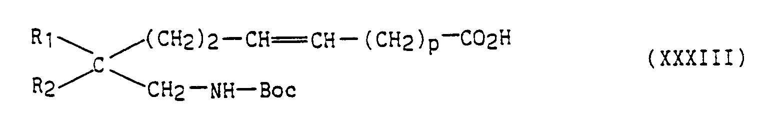 in der R₁, R₂ und p die bezüglich der Formel (I') angegebenen Bedeutungen besitzen,
von deren Aminfunktion man die Schutzgruppe mit Chlorwasserstoff-haltigem Ether abspaltet und dann den gleichen Reaktionen unterwirft, wie sie für die Umwandlung der Verbindung (XXVII) in die Verbindung (XXVIII) beschrieben sind,
so daß man die Verbindung der Formel (I/f) erhält, einem Sonderfall der Verbindungen der Formel (I): 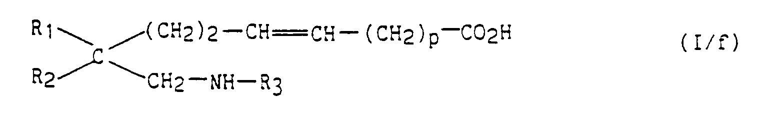 in der R₁, R₂, R₃ und p die bezüglich der Formel (I') angegebenen Bedeutungen besitzen,
- welche Verbindungen der Formeln (I/e) oder (I/f) man gewünschtenfalls durch katalytische Hydrierung reduziert zur Bildung der Verbindungen der Formeln (I/e₁) oder (I/f₁), einem Sonderfall der Verbindungen der Formel (I): 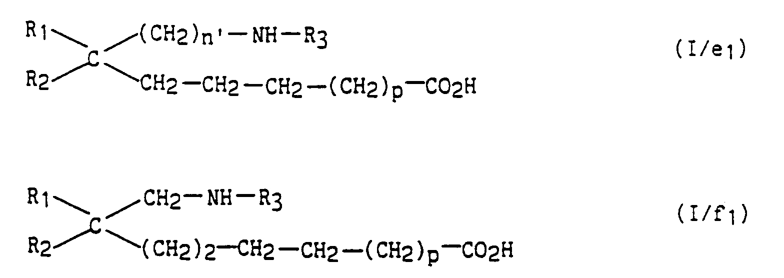 in denen R₁, R₂, p und n' die bezüglich der Formel (I) angegebenen Bedeutungen besitzen,
- welche Verbindungen der Formeln (I/e), (I/e₁), (I/f) oder (I/f₁):
- man mit Hilfe klassischer Reinigungsverfahren reinigt,
- gegebenenfalls mit Hilfe klassischer Trennungsverfahren in die Isomeren auftrennt und
- gewünschtenfalls mit Hilfe einer pharmazeutisch annehmbaren Säure oder Base in ihre Additionssalze umwandelt.

12. Pharmazeutische Zubereitungen enthaltend als Wirkstoff mindestens eine Verbindung nach einem der Ansprüche 1 bis 9 allein oder In Kombination mit einem oder mehreren inerten, nichttoxischen, pharmazeutisch annehmbaren Trägermaterialien.

13. Pharmazeutische Zubereitung nach Anspruch 8 enthaltend mindestens einen Wirkstoff nach einem der Ansprüche 1 bis 9 zur Verwendung als Antagonisten von Rezeptoren des Thromboxans A₂ oder als Inhibitoren der Thromboxan-A₂-Synthase.
